(19) Europäisches Patentamt
European Patent Office
Office européen des brevets

(11) **EP 2 781 518 A2**

(12) **EUROPEAN PATENT APPLICATION**
published in accordance with Art. 153(4) EPC

(43) Date of publication:
**24.09.2014 Bulletin 2014/39**

(21) Application number: **12848204.9**

(22) Date of filing: **01.11.2012**

(51) Int Cl.:
***C07D 471/04*** *(2006.01)* ***A61K 31/407*** *(2006.01)*
***A61P 25/00*** *(2006.01)*

(86) International application number:
**PCT/RU2012/000894**

(87) International publication number:
**WO 2013/070117 (16.05.2013 Gazette 2013/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.11.2011 RU 2011145513**

(71) Applicant: **Federalnoe Gosudarstvennoe Bydzhetnoe Uchrezhdenie Nauki Institut Fiziologicheski Aktivnikh Veschestv Rossiiskoi Akademii Nauk (IFAV RAN)**
**Moskovskaya obl. 142432 (RU)**

(72) Inventors:
- **BACHURIN, Sergey Olegovich**
  **Moskovskaya obl. 142432 (RU)**
- **USTUGOV, Aleksey Anatolyevich**
  **Moskovskaya obl. 142432 (RU)**
- **NINKINA, Natalya Nikolaevna**
  **Moskovskaya obl. 142432 (RU)**
- **SOKOLOV, Vladimir Borisovich**
  **Moskovskaya obl. 142432 (RU)**
- **AKSINENKO, Aleksey Yuryevich**
  **Moskovskaya obl. 142432 (RU)**
- **SHELKOVNIKOVA, Tatyana Aleksandrovna**
  **Moskovskaya obl. 142432 (RU)**
- **BOLKUNOV, Aleksey Viktorovich**
  **Moskovskaya obl. 142432 (RU)**

(74) Representative: **King, Lawrence**
**A.A. Thornton & Co.**
**10 Old Bailey**
**London EC4M 7NG (GB)**

(54) **FLUORINE-CONTAINING 5-[2-(PYRID-3-YL)-ETHYL]-2,3,4-TETRAHYDRO-1H-PYRIDO[4,3-B] INDOLES AS AGENTS FOR REDUCING UNCONTROLLED PROTEIN AGGREGATION**

(57) The claimed fluorine-containing substituted 5-[2-(pyrid-3-yl)-ethyl]-2,3,4-tetrahydro-1H-pyrido[4,3-b]indoles and hydrochlorides and hydrobromides thereof, which have the general formula (I), as agents for reducing uncontrolled protein aggregation in the nervous system, the pharmacological agent based thereon and the method for using same relate to the field of medicine and solve the problem of increasing the range of agents for combating the development of destructive processes in the central and peripheral nervous system.

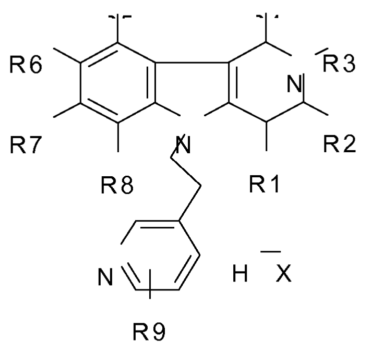

(I)

where $R_1 = R_2 = R_4$ = H, Me; or $R_4 + R_2 = -CH_2-CH_2-$; $R_3$ = H, $(C_1-C_6)$alkyl, $(C_2-C_6)$alkenyl, $(C_3-C_6)$alkynyl, $(C_1-C_6)$alkoxy $(C_1-C_6)$alkyl, [F, Cl, Br, $NO_2$, $(C_1-C_6)$alkyl, $(C_1-C_6)$ alkoxy, acyl, $(C_1-C_6)$alkylsulfonyl, $(C_1-C_6)$alkoxycarbonyl - substituted] aryloxy $(C_1-C_6)$alkyl, $(C_3-C_7)$cycloalkyl, [F, Cl, Br, $NO_2$, $(C_1-C_6)$alkyl, $(C_1-C_6)$alkoxy, acyl, $(C_1-C_6)$ alkylsulfonyl, $(C_1-C_6)$alkoxycarbonyl - substituted] aryl, heteryl, $(C_1-C_6)$alkylsulfonyl, $(C_1-C_6)$alkoxycarbonyl, $(C_1-C_6)$ acyl, [F, Cl, Br, $NO_2$, $(C_1-C_6)$alkyl, $(C_1-C_6)$alkoxy, acyl, $(C_1-C_6)$alkylsulfonyl, $(C_1-C_6)$alkoxycarbonyl - substituted] aroyl, heteroyl, N,N-dialkylcarbamoyl, N,N-dialkylaminosulfonyl;
$R_5, R_6, R_7, R_8$ = H, F, Cl, Br, CN, OH, $CF_3$, $CF_3O$, $CHF_2O$, $NO_2$, $(C_1-C_6)$alkyl, $(C_3-C_7)$cycloalkyl, $(C_1-C_6)$alkoxy, [F, Cl, Br, $NO_2$, $(C_1-C_6)$alkyl, $(C_1-C_6)$alkoxy, acyl, $(C_1-C_6)$ alkylsulfonyl, $(C_1-C_6)$ alkoxycarbonyl - substituted] aryl, [F, Cl, Br, $NO_2$, $(C_1-C_6)$ alkyl, $(C_1-C_6)$alkoxy, acyl, $(C_1-C_6)$ alkylsulfonyl, $(C_1-C_6)$ alkoxycarbonyl - substituted] aryloxy, (Me, Cl, Br - substituted) pyridyl, $(C_1-C_6)$alkylsulfonyl, $(C_1-C_6)$ alkoxycarbonyl, $(C_1-C_6)$acyl, [F, Cl, Br, $NO_2$, $(C_1-C_6)$alkyl, $(C_1-C_6)$alkoxy, acyl, $(C_1-C_6)$alkylsulfonyl, $(C_1-C_6)$alkoxycarbonyl - substituted] aroyl, N,N-dialkyl-

EP 2 781 518 A2

carbamoyl, N,N-dialkylaminosulfonyl;

$R_9$ = F, 2F, $CHF_2$, $CC1F_2$, $CF_3$.; X = nothing, Cl. Br.

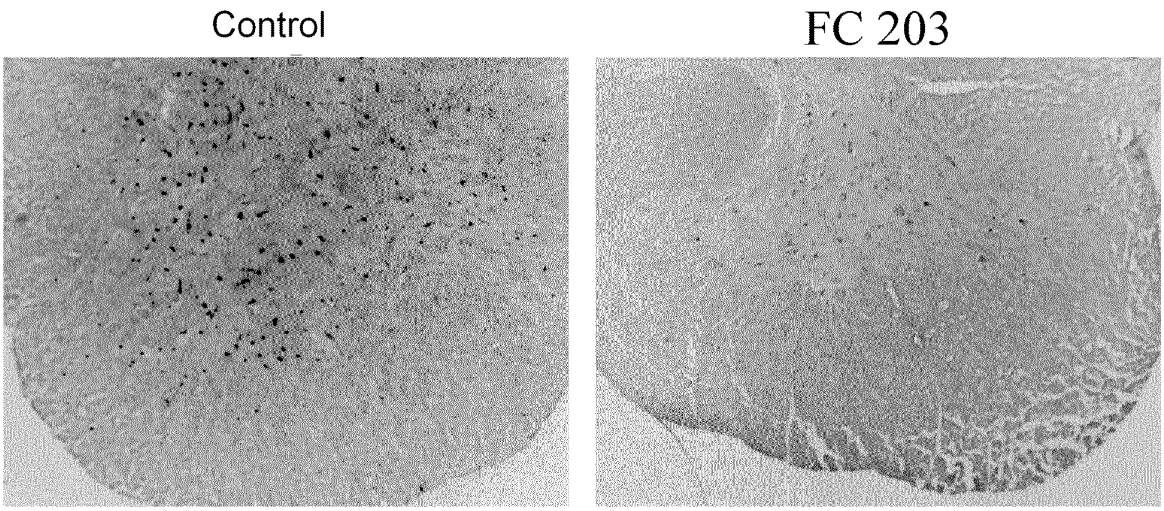


FIG. 1

## Description

The invention relates to the use of chemical compounds in the field of medicine and can be used as an effective agent in the manufacture of pharmacological preparations for the prevention and treatment of diseases associated with uncontrolled protein aggregation.

Uncontrolled aggregation of certain proteins is a key factor in the development of neurodegenerative processes underlying pathogenesis of multiple neurodegenerative diseases. Accumulation, in the nervous system, of various intermediates (oligomers, protofibrills) and final (fibrillar and amorphous deposits) products, many of which have cytotoxic properties, results in functional disorders and, finally, in the neuron death. Such type of pathology, known as proteinopathy, combines in one group neurological disorders with essentially different clinical pathological manifestations. This group of diseases includes neurodegenerative disorders, which are widely distributed and currently incurable, such as Alzheimer's disease (AD), Parkinson disease (PD), amyotrophic lateral sclerosis (ALS), frontotemporal degeneration (FTD), Huntington's chorea and prion diseases. Up to the present day the treatment of proteinopathies is preferably symptomatic since effective agents acting directly to the pathological process underlying the disease still have not been developed.

At present, uncontrolled protein aggregation is considered to be one of the important targets for the development of a novel generation of therapeutic agents allowing the modification of the processes underlying the development of neurodegeneration, thereby retarding the development of the disease and even arresting the pathological process [Jacobsen J.S., Reinhart P., Pangalos M.N. Neuro R.X., 2005, vol. 2(4), pp.612-626; Walker L.C., Ibegbu C.C., Todd C.W., Robinsona H.L., Juckere M., IIlf H.L., Gandyg S., Biochemical Pharmacology, 2005, vol. 69, pp.1001-1008; Christensen D.D, CNS Spectrums, 2007, vol. 12, pp.113-123].

It is known that a derivative of hydrogenated pyrido[4,3-b]indoles, in particular, gamma-carboline - Dimebon - modulates the molecular cellular processes of formation and/or elimination of pathological protein depositions of amyloid-like inclusions formed by aggregation-prone gamma-synuclein protein in the tissues of the nervous system of model animals (S.O. Bachurin, A.A. Ustyugov, O. Peters, T.A. Shelkovnikova, V.L. Buchman, N.N. Ninkina, Doklady of the Russian Academy of Sciences, 2009, 428, 262-265).

However, the pharmacokinetic properties of Dimebon do not attribute to the sufficient broad spectrum of action of its derivatives.

The claimed invention addresses the issue of broadening the spectrum of agents involved in modulation of uncontrolled protein aggregation in the nervous system which subsequently leads to the development of degenerative processes in the central and peripheral nervous systems.

The use of fluorine-containing substituted 5-[2-(pyrid-3-yl)-ethyl]-2,3,4-tetrahydro-1H-pyrido[4,3-b]indoles, and respective hydrochlorides and hydrobromides whit the general formula (I) as effective agents for reducing the uncontrolled protein aggregation in the nervous system.

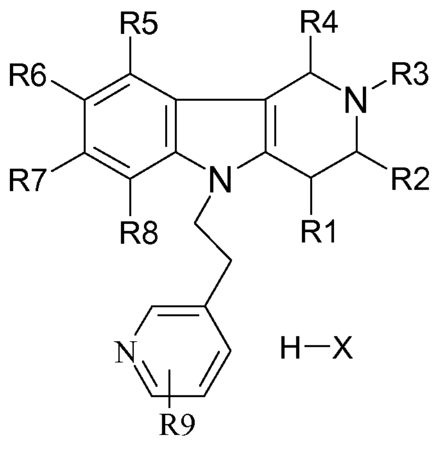

(I),

Where:

$R_1$=$R_2$=$R_4$ are H or Me; or $R_1$ + $R_2$ is $-CH_2-CH_2-$;

$R_3$ is H, ($C_1$-$C_6$) alkyl, ($C_2$-$C_6$)alkenyl, ($C_3$-$C_6$)alkynyl, ($C_1$-$C_6$)alkoxy($C_1$-$C_6$)alkyl, [F, Cl, Br, $NO_2$, ($C_1$-$C_6$)alkyl, ($C_1$-$C_6$)alkoxy, acyl, ($C_1$-$C_6$)alkylsulfonyl, ($C_1$-$C_6$)alkoxycarbonyl-substituted] aryloxy ($C_1$-$C_6$) alkyl, ($C_3$-$C_7$)cycloalkyl, [F, Cl, Br, $NO_2$, ($C_1$-$C_6$)alkyl, ($C_1$-$C_6$)alkoxy, acyl, ($C_1$-$C_6$)alkylsulfonyl, ($C_1$-$C_6$)alkoxycarbonyl - substituted]aryl, heteroaryl, ($C_1$-$C_6$)alkylsulfonyl, ($C_1$-$C_6$)alkoxycarbonyl, ($C_1$-$C_6$)acyl, [F, Cl, Br, $NO_2$, ($C_1$-$C_6$)alkyl, ($C_1$-$C_6$)alkoxy, acyl, ($C_1$-$C_6$)alkylsulfonyl, ($C_1$-$C_6$)alkoxycarbonyl - substituted]aroyl, heteroyl, N,N-dialkylcarbamoyl,

N,N-dialkylaminosulfonyl;
$R_5$, $R_6$, $R_7$, $R_8$ = H, F, Cl, Br, CN, OH, $CF_3$, $CF_3O$, $CHF_2O$, $NO_2$, $(C_1-C_6)$alkyl, $(C_3-C_7)$cycloalkyl, $(C_1-C_6)$alkoxy, [F, Cl, Br, $NO_2$, $(C_1-C_6)$alkyl, $(C_1-C_6)$alkoxy, acyl, $(C_1-C_6)$alkylsulfonyl, $(C_1-C_6)$alkoxycarbonyl- substituted] aryl, [F, Cl, Br, $NO_2$, $(C_1-C_6)$alkyl, $(C_1-C_6)$alkoxy, acyl, $(C_1-C_6)$alkylsulfonyl, $(C_1-C_6)$alkoxycarbonyl - substituted]aryloxy, (Me, Cl, Br-substituted)pyridyl, $(C_1-C_6)$alkylsulfonyl, $(C_1-C_6)$alkoxycarbonyl, $(C_1-C_6)$acyl, [F, Cl, Br, $NO_2$, $(C_1-C_6)$alkyl, $(C_1-C_6)$alkoxy, acyl, $(C_1-C_6)$alkylsulfonyl, $(C_1-C_6)$alkoxycarbonyl - substituted] aroyl, N,N-dialkylcarbamoyl, N,N-dialkylaminosulfonyl;
R9 = F, 2F, $CHF_2$, $CClF_2$, $CF_3$;
X is absent or is Cl or Br.

Another aspect of the invention is a pharmacological agent for reducing uncontrolled protein aggregation in the nervous system, the agent comprising an active agent and a pharmaceutically acceptable carrier, characterized in that as the active agent the pharmacological agent comprises an effective amount of the compound of formula (I).

Another aspect of the invention is a method for reducing uncontrolled protein aggregation in the nervous system, comprising administering to a patient a pharmacological agent comprising an effective amount of the compound of formula (I) in a dose of from 0.05 to 1.5 mg/kg of body weight once a day over a period of time required for a therapeutic effect.

It is known that fluoridated derivatives of pyrido[4,3-b]indoles are used as potential agents for the treatment of neurodegenerative disorders such as Alzheimer's disease (AD), Parkinson disease (PD), Amyotrophic lateral sclerosis (ALS), schizophrenia, multiple sclerosis and a number of others due to the ability of these derivates to bind to histamine, adrenergic, dopamine, serotonin, imidazoline AMPA- and aminergic protein-coupled receptors, as well as the potency of these compounds to have an effect on the reuptake of $Ca^{2+}$ in rat brain synaptosomes and to improve memory in rats treated with scopolamine (application: WO 2009/038764, A1, C07D471/04 (2006/01), 26.03.2009; application WO 2009/055828, A1, C07D471/04 (2006/01), 30.04.2009).

During our studies, we unexpectedly found that chronic use of fluorine-containing substituted 5-[2-(pyrid-3-yl)-ethyl]-2,3,4-tetrahydro-1H-pyrido[4,3-b]indoles of formula (I) in animals with overexpression of aggregation-prone proteins caused by a genetic modification, in particular gamma-synuclein protein and tau protein, leads to a significant reduction in the aggregation of these proteins, suppression of astrogliosis as well as an increase in the amount of surviving neurons.

A technical effect that can be obtained when embodying the invention is in widening of the range of pharmaceutical agents based thereon.

Thus, finding new properties of known compounds outlined in formula (I) allows the use thereof not only for more effective treatment of Alzheimer's disease (AD), Parkinson disease (PD), Amyotrophic lateral sclerosis (ALS), Frontotemporal degeneration (FTD), Huntington's disease, but also for the prevention and treatment of broader range of neurodegenerative diseases, in particular, progressive supranuclear palsy, corticobasal degeneration, Pick's syndrome, and prion diseases, which are directly associated with uncontrolled protein aggregation in the nervous system.

Tables 1 and 2 summarize yields, melting points and $^1H$ and $^{19}F$ NMR spectra of claimed classes of compounds.

Fig.1 shows immunohistochemical staining of spinal cord sections of 12-month old homozygous Thy1m$\gamma$SN transgenic mice with antibodies to gamma-synuclein, for animals that were not treated with a compound (control) and for animals that were treated with a compound from the age of three-month; 100x magnification.

Fig.2 shows results of the quantification of gamma-synuclein-positive aggregates in spinal cord sections of 12-month old homozygous transgenic mice of the control group and animals treated with FC-203 compound (fluorine-containing carboline) of formula 9. The density of aggregates was calculated per unit area.

Fig.3 shows immunohistochemical staining of spinal cord sections of 4- and 5-month old homozygous P301S transgenic mice with antibodies against tau protein, for animals that were not treated with a compound (control) and for animals that were treated with FC-203 from the age of five-weeks; 400x magnification.

Fig.4 shows results of the quantification of tau-positive aggregates in spinal cord sections of 4- and 5-month old homozygous transgenic mice in the control and experimental groups (n=3, mean value $\pm$ SEM).

Fig.5 shows immunohistochemical staining of glial fibrillar acid protein (GFAP) in sections of spinal cord in 12-month old homozygous Thy1m$\gamma$SN transgenic mice in the control and experimental groups; 400x magnification.

Fig.6 shows immunohistochemical staining of GFAP in sections of spinal cord in 4- and 5-month old homozygous P301S transgenic mice in the control and experimental groups.

Fig.7 shows the mean latency to fall from a rotarod for Thy1m$\gamma$SN mice in FC-203 treated group (squares) administered from the age of one month compared to the control group (circles).

Fig.8 shows the mean latency to fall from a rotarod for P301S mice in FC-203 treated group (squares) administered from the age of one month compared to the control group (circles).

Fig.9 shows the mean latency to fall from an inverted grid for Thy1m$\gamma$SN mice in FC-203 treated group (squares) administered from the age of one month compared to the control group (circles).

Fig.10 shows the mean latency to fall from an inverted grid for P301S mice in FC-203 treated group (squares) administered from the age of one month old compared to the control group (circles).

Fig. 11.shows immunohistochemical detection of the aggregation-prone of human FUS protein in SH-SY5Y neuroblastoma cells with antibodies to the C-terminal epitope of this protein.

Fig. 12.shows immunohistochemical detection of an aberrant human TDP-43 protein which characterized by an increased aggregation in SH-SY5Y neuroblastoma cells with antibodies to the C-terminal epitope.

**Synthesis of compounds**

Compounds of formula (I) - fluorine-containing substituted 5-[2-(pyrid-3-yl)-ethyl]-2,3,4-tetrahydro-1H-pyrido[4,3-b]indoles, and respective hydrochlorides and hydrobromides, according to the invention are obtained by methods known in the prior art for preparing similar compounds from substituted 2,3,4-tetrahydro-1H-pyrido[4,3-b]indoles and fluorine-containing 3-vinylpyridine compounds. Reaction of 2,3,4-tetrahydro-1H-pyrido[4,3-b]indoles and a mixture of fluorine-containing 3-vinylpyridine compounds is carried out by heating an equimolar mixture of reagents in dimethyl sulfoxide (DMSO) at 135-140°C for from 8 to 10 hours in the presence of catalytic amounts of cesium fluoride resulting in the formation of fluorine-containing substituted 5-[2-(pyrid-3-yl)-ethyl]-2,3,4-tetrahydro-1H-pyrido[4,3-b]indoles (I), the treatment of which with hydrochloric or hydrobromic acid leads to the corresponding hydrochlorides and hydrobromides (scheme 1).

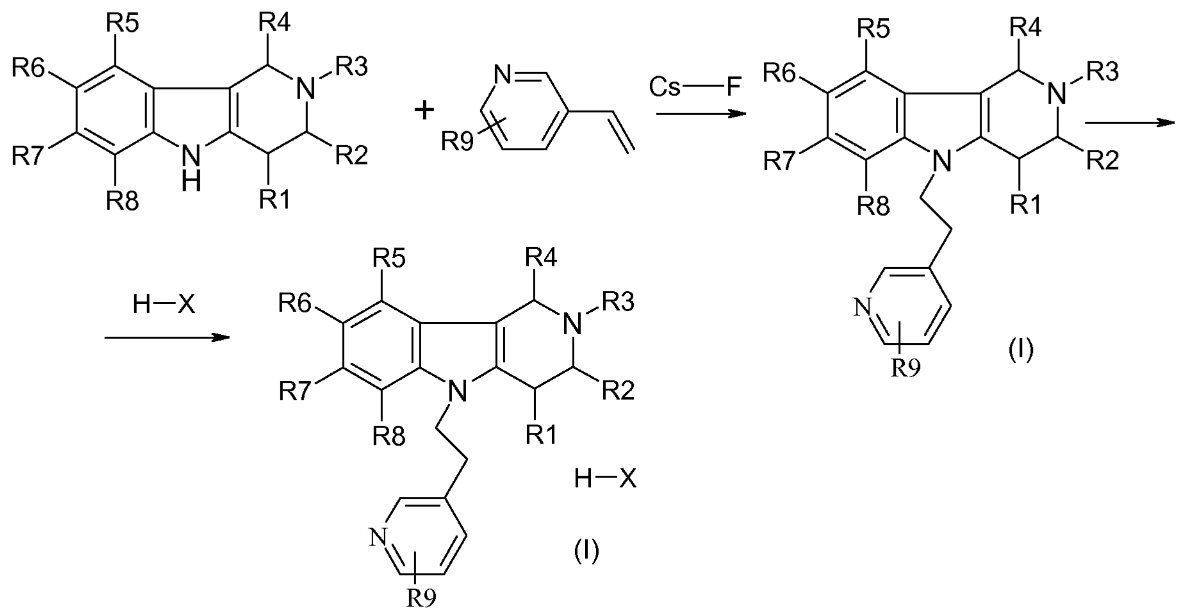


Scheme 1

Feasibility of the invention with achieving the claimed intended purpose is supported by, but not limited to, the following examples.

**Example 1. <u>Synthesis of fluorine-containing substituted 5-[2-(pyrid-3-yl)-ethyl]-2,3,4-tetrahydro-1H-pyrido[4,3-b]indoles (general method)</u>**

1 mmol of 2,3,4-tetrahydro-1H-pyrido[4,3-b]indole, 1 mmol of fluorine-containing 3-vinylpyridine, 200 mg of CsF, and 5 mg of hydrochinone in 1.5 ml of DMSO were heated under stirring from 135 to 140°C for 8 hours. DMSO and vinylpyridine were removed under vacuum (3 mm Hg), and the product was extracted from the residue with methylene chloride. Methylene chloride was evaporated, and the residue was purified by chromatography on silica gel (60 mesh, eluent: methanol/chloroform = 1/5).

Yields, melting points, and data of $^{1}H$ and $^{19}F$ NMR spectra of compounds 1-7 and 23-29 are in Tables 1 and 2.

**Example 2. Synthesis of hydrochlorides of fluorine-containing substituted 5-[2-(pyrid-3-yl)-ethyl]-2,3,4-tetrahydro-1H-pyrido[4,3-b]indoles (general method)**

0.1 ml of concentrated hydrochloric acid was added to a suspension of a base in 5 ml of water and heated until the base was completely dissolved. Water and hydrochloric acid were evaporated under vacuum, and the resulting residue was recrystallized from 50% ethanol.

Yields, melting points, and data of $^1H$ and $^{19}F$ NMR spectra of compounds 8-21 and 30-36 are in Tables 1 and 2.

**Example 3. Synthesis of hydrobromides of fluorine-containing substituted 5-[2-(pyrid-3-yl)-ethyl]-2,3,4-tetrahydro-1H-pyrido[4,3-b]indoles (general method)**

0.1 ml of concentrated hydrobromic acid was added to a suspension of a base in 5 ml of water and heated until the base was completely dissolved. Water and hydrobromic acid were evaporated under vacuum, and the resulting residue was recrystallized from 50% ethanol. Yields, melting points, and data of $^1H$ and $^{19}F$ NMR spectra of compound 22 are in Tables 1 and 2.

**Biological activity of the obtained compounds**

The feasibility of the invention with achieving of the claimed intended purpose is exemplified using test compound FC-203.

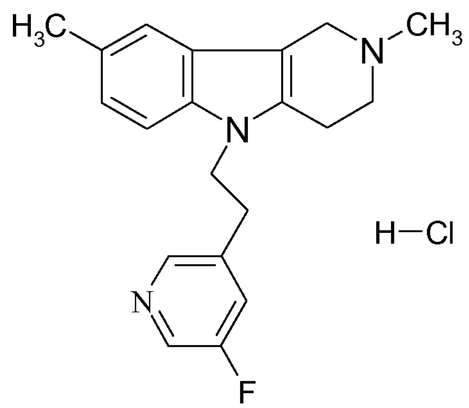

Biological properties of this compound were studied in proteinopathies models both *in vitro* and *in vivo.* Human SH-SY5Y neuroblastoma cells with an ectopic expression of amyloidogenic TDP-43 and FUS proteins were used as *in vitro* models allowing the reproduction of key mechanisms of the molecular pathology associated with the disturbance in the metabolism of these proteins.

This approach is complementary to the studies in transgenic animals which is focused on discovery of molecular mechanisms and development of neurodegenerative changes. In addition, it is also possible to use these animal models for selection of therapeutic agents that have a direct effect on the mechanisms of uncontrolled protein aggregation. Mutant forms of TDP-43 and FUS proteins which tend to form pathological inclusions in the nervous tissue were selected as amyloidogenic proteins. It was previously shown that TDP-43 and FUS proteins are involved in the regulation of processing, transportation and metabolism of RNAs. Dysfunction of these proteins can cause the development of proteinopathy. Recently, point mutations in the TDP-43 and FUS genes were discovered in patients with hereditary and idiopathic forms of some variants of Amyotrophic lateral sclerosis (ALS) and Frontotemporal degeneration with ubiquitin inclusions (FTD-U) resulting in replacement of an amino acid sequence in the coding region these genes. Such type of molecular pathology can be reproduced in cellular model systems.

It was shown overexpression of these model proteins in human SH-SY5Y neuroblastoma cells leads to uncontrolled aggregation of aberrant forms of these proteins was, which was also accompanied by the formation and accumulation of insoluble pathological inclusions. The same molecular pathology could be reproduced in cellular systems specific for each mutation of TDP-43 or FUS genes, leading to the damage in the structure of encoded proteins.

Biological action of FC-203 (formula 9) was studied on two different types of proteinopathy. Thy1mgSN and P301S transgenic lines of mice were used as *in vivo* models. Thy1mgSN transgenic mice generated on C57B16J background and it is characterized by a high level of ectopic expression of human gamma-synuclein in the tissues of the central nervous system. The transgene expression was under the control of the neuron-specific promoter Thy-1. This line is commercially available through the Jackson Laboratory catalogue (http://iaxmice.iax.org/strain/Q08843.html).

It is characterized by a progressive accumulation of aggregation-prone gamma-synuclein protein, formation of intracellular pathological amyloid-like deposits and its accumulation in the tissues of different parts of the central nervous system, which, in turn, promotes the reproduction of the key pathogenic mechanisms of proteinopathy.

Another line of transgenic mice was P301S (full name of the line according to the Jacksons Laboratory database - B6;C3-Tg(Prnp-MAPT*P301S)PS19Vle/J) and it is characterized by overexpression of aberrant human tau protein. The transgene expression is under the control of the prion protein promoter *Prnp* in the tissues of the central nervous system of model animals and it leads to the accumulation of hyperphosphorilated tau protein that forms neurofibrillary tangles, which are characteristic histological structures observed in tauopathies such as Alzheimer's disease, progressive supranuclear palsy, corticobasal degeneration and Pick's disease. Thus, P301S reproduces key mechanisms of proteinopathies associated with uncontrolled protein aggregation.

Experimental and control groups of animals were formed from the offspring produced by crossing heterozygous parents with the wild-type mice. Cohorts of males that were homozygous for the transgenic allele were used. The housing conditions were the same for the control and experimental groups and remained unchanged during the whole span of experimental program: a 12-hour light cycle, 22°C, free access to food and water. The test compound FC-203 was dissolved in drinking water (70 μg/ml) and this solution was used as the only source of drinking water in the experimental group of mice, the access to the drinking solution comprising compound FC-203 was unlimited. The drinking solution was changed 3 times per week. Daily water intake of the animals was recorded and corresponded to the physiological standard for the liquid intake and it was on average 5 ml per mouse weighing 30 grams.

Daily intake of the drinking solution containing FC-203 did not differ from the daily intake of drinking water and it was also around 5 ml per mouse weighing 30 grams. Correspondingly, in terms of animal body weight, a daily intake of the compound was 11.7 mg/kg.

$$70\frac{\mu g}{mL} \times 5\ mL = 350\ \mu g\ per\ day\ for\ one\ mouse$$

$$\frac{350\ \mu g}{30\ g} \times \frac{1000\ g}{1\ kg} \times \frac{1\ mg}{1000\ \mu g} = 11.7\ \frac{mg}{kg}$$

based on weight

**Example 4. <u>Determination of FC-203 effect on the composition of pathological amyloid-like inclusions formed by gamma-synuclein protein in the affected parts of the nervous system in Thy1mγSN mice</u>**

The compound was administered to the experimental group of mice starting from the age of 12-week for 9 months. The control group was not administered with the compound. Spinal cord tissues were taken for the analysis both from the control and experimental groups after drug-induced euthanasia by intraperitoneal administration of a lethal dose of sodium pentobarbital (Euthatal). The spinal cords were removed and divided into three separate regions: cervical, dorsal and lumbar which were fixed separately. The spinal cords were fixed in a cold Carnoy solution (60% ethanol, 30% chloroform and 10% glacial acetic acid) overnight at 4°C for further staining with specific agents to amyloid deposits. Tissues used for immunohistochemical analysis were fixed in 4% paraformaldehyde in phosphate buffer (PBS). Then, the samples were washed with distilled water (two times, 1 hour) and the tissues were sequentially dehydrated with 75% ethanol (15 min), 96% ethanol (two times, 5 and 10 min), 100% ethanol (two times, 10 min), ethanol/chloroform (1:1, 30 min), and chloroform (1 hour) at room temperature, and then incubated in chloroform overnight at 4°C, after that the samples were embedded in paraffin. The cross-sections of the spinal cord were analyzed on the number and the distribution of aggregates.

**Preparation of histological sections.** 8 μm thick sections were cut at a Leica RM2265 rotary microtome and mounted on SuperFrost slides (BDH, Poole, UK) for further staining with Congo Red, or on Gold Seal Slides (Gold Seal Products, UK) for further immunostaining. The sections were deparaffinized in xylene (two times, 5 min) and rehydrated through graded decreasing concentrations of alcohols (ethanol, room temperature).

**Congo Red staining and counting of amyloid deposits.** After deparaffinization and rehydratation through graded decreasing concentrations (100%, 90%, 70%) of alcohols (ethanol), the sections were washed two times with distilled water (5 min). The sections were stained with a 0.5% Congo Red solution (Sigma, USA) in 50% alcohol for 7 min at room temperature. The staining was fixed in 0.2% KOH in 80% alcohol for 1 min. Then, the sections were washed with water and air-dried. Sections were mounted with a drop of Immu-Mount (Thermo Electron Corporation, USA) and covered with a coverslide (50x24 mm), dried in a dry air sterilizer at 37°C or at room temperature. The results were analyzed and recorded with a fluorescent microscope Leica DMI 4000B. Photomicrographs were made with a Leica

DFS 490 camera and using Leica Application Suite version 2.8.1 software (Leica Microsystems, Germany). The number of stained amyloid deposits was counted in the gray matter sample with area of 0.1 $mm^2$ using the ImageJ software (http://rsbweb.nih.gov/ij/). Statistical analysis was carried out using the Microsoft Office 2003 Excel software package (Microsoft Corp., USA).

The results of the experiment in Fig. 1 and 2 show that systematic administration of compound FC-203 significantly decreases the number of amyloid deposits in the spinal cord tissues of Thy1mγSN transgenic mice. It could be attributed by two possible mechanisms: FC-203 can either prevent the formation of new aggregates or have an effect on the stability of already pre-formed inclusions, for example, through the activation of cell's own defense mechanisms aimed to clean the inside functional space from aberrant proteins and fibrillar structures.

**Example 5. <u>Determination of FC-203 effect on the number of histopathological amyloid-like inclusions in the tissues of the spinal cord of P301S mice</u>**

The effect of FC-203 on the number of human tau protein aggregates was studied in a mice line overexpressing phosphorylated mimic of tau protein, which is characterized by early neurological pathology associated with tau protein deposits in neurons. The experimental group of P301S transgenic mice was treated with FC-203 starting from the age of 5-week when the formation of neurofibrillary tangles was not detected histologically. Mice in the experimental groups were treated for 15 or 20 weeks (up to 4- or 5-month old, respectively). The results of the analysis of the number of pathological aggregates of tau protein in spinal cords of P301S transgenic mice (Fig. 3 and 4) indicate that a long-term administration of the FC-203 which had started on the pre-somatic stage of proteinopathy, leads to a statistically significant reduction in the number of tau-positive deposits in the spinal cord neurons of the transgenic animals.

Thus, FC-203 effectively reduces the formation of deposits of human tau protein, which is directly related with its anti-aggregation properties *in vivo.*

**Example 6. <u>Determination of FC-203 effect on the process of astrogliosis in animal neurons with an enhanced synthesis of gamma-synuclein</u>**

The effect of the testing compound on the processes associated with reactive astrogliosis as a marker of inflammatory reaction intrinsic to the diseases with uncontrolled protein aggregation was studied based on the levels of expression of glial fibrillary acid protein (GFAP) which is often used a marker for neuroinflammatory response in nervous tissues. We used both lines of transgenic mice Thy1mγSN and P301S. To assess the efficacy of the compound we relied on the rule that smaller was the inflammatory response in tissues (which was measured by activated astrocytes with specific color), the more effective was agent for suppression of inflammation. This experiment was conducted with sections of the spinal cord prepared by the method described in Example 5. Then, the sections were incubated with primary polyclonal rabbit antibodies to GFAP (DAKO, USA) in blocking buffer (dilution 1:1000) at 4°C overnight.

Data in Fig.5 shows the effect of FC-203 on the astrogliosis in animal neurons with an enhanced expression of gamma-synuclein. As a result, a long-term administration of the compound significantly decreases the level of activated astrocytes in animals with gamma-synucleinopathy.

Thus, FC-203 effectively inhibits neural-specific inflammation that usually is associated with uncontrolled protein aggregation in the tissues of the nervous system in animals.

**Example 7. <u>Determination of FC-203 effect on the development of astrogliosis in animal neurons with an enhanced synthesis of hyperphosphorylated tau protein</u>**

The experimental conditions were the same as disclosed in Example 6.

The number of detected activated astrocytes in P301S mice with a pathology caused by the aggregation of hyperphosphorylated human tau protein increases with the progression of the neurodegenerative process and corresponds to the stage of the development of proteinopathy: in Thy1mγSN mouse the symptomatic stage of gamma-synucleinopathy becomes detectable from 6 months, whereas the neuronal symptoms caused by taupathy in P301S mice is developed far earlier. Correspondingly, astrogliosis is also initiated in P301S mice much earlier than in Thy1mγSN animals. The use of the FC-203 significantly reduces the intensity of astrogliosis in two animal models of symptomatic stages of proteinopathy.

Based on our result (Fig. 6), the testing compound significantly reduces the level of astrogliosis caused by uncontrolled aggregation of tau protein. This is direct evidence that FC-203 effectively inhibits inflammation associated with the process of uncontrolled protein aggregation in the tissues of the nervous system in animals.

**Example 8. Determination of FC-203 effect on the progression of symptoms associated with uncontrolled aggregation of gamma-synuclein in the nervous system of transgenic animals: analysis of balance and coordination on a rotating rod**

The motor function of Thy1mγSN transgenic mice was tested on their ability to maintain balance on the rotating rod under continuous acceleration. Each test on a rotating rod was conducted for 5 min; the speed of the rotation was increased for this period of time from 4 to 40 rpm. The latency to fall when the animal is no longer able to remain on the rotating rod was recorded for each mouse. The test was repeated three times for each animal with 30-min rest intervals and the mean latency to fall was calculated.

The animals were tested on the rotating rod under continuous acceleration. The mean latency to fall from the rotating rod are given for the experimental group of animals treated with FC-203 from the age of one month (squares) compared to the non-treated control group (circles).

Fig.7 indicates that the test compound FC-203 significantly inhibits the development of the clinical symptoms of neurodegenerative process caused by gamma-synucleinopathy since the balance and coordination of the transgenic mice treated with the testing compound were statistically better than those in the control untreated mice.

**Example 9. Determination of FC-203 effect on the progression of symptoms associated with uncontrolled aggregation of hyperphosphorylated tau protein in the nervous system of transgenic animals: analysis of balance and coordination on a rotating rod**

The motor function of P301S transgenic mice was tested according to their ability to maintain balance on a rotating rod under continuous acceleration. Each test on the rotating rod was conducted for 5 min; the speed of the rotation was increased for this period of time from 4 to 40 rpm. The latency to fall when the animal is no longer able to remain on the rotating rod was recorded for each mouse. The test was repeated three times for each animal with 30-min rest intervals and the mean latency to fall was calculated.

The animals were tested on the rotating rod under continuous acceleration. Fig.8 indicates the mean latency to fall for the control (circles) and experimental FC-203 (squares) groups, wherein the experimental animals were treated with testing compound from the age of one month. The obtained data show that the testing compound significantly inhibits the development of clinical symptoms of taupathy.

Thus, FC-203 effectively reduces balance and coordination disturbance in the P301S transgenic mice, caused by tau protein aggregation.

**Example 10. Determination of FC-203 effect on the progression of symptoms associated with uncontrolled aggregation of gamma-synuclein in the nervous system of transgenic mice: assessment of animal ability to hold onto an inverted grid**

The progression of neurodegenerative processes caused by gamma-syncleinopathy in Thy1mγSN transgenic mice leads to a detectable motor function failures associated with nerve degeneration and development of muscle weakness (the animals lose their ability to hold their body on an inverted grid). The ability of the animals to hold their body on an inverted grid was evaluated continuously every month starting from the age of three month for both groups of animals. As a result the mean latency (in minutes) to fall was recorded for each group.

Fig.9 indicates the mean latency for the control group (circles) and experimental FC-203 group (squares), where the mice in the experimental group were administered the testing compound from the age of three month.

The obtained data show that the testing compound significantly inhibits the development of clinical symptoms caused by anomalous limbs innervation in tested animals. Thus, FC-203 has a pronounced effect on the progression of gamma-syncleinopathy in transgenic mice.

**Example 11. Determination of FC-203 effect on the progression of symptoms associated with uncontrolled aggregation of hyperphosphorylated tau protein in the nervous system of transgenic animals: analysis of animal ability to hold their body on an inverted grid**

The ability of the animals to hold their body on an inverted grid was evaluated every month, from the age of two month both for the treated and untreated control groups.

The mean latency obtained for each group of mice in the control (circles) and experimental (squares) groups were calculated, wherein the experimental animals were treated with FC-203 from the age of one month.

Fig.10 indicates that the test compound significantly inhibits the development of clinical symptoms caused by anomalous limb innervation in mice caused by taupathy. Thus, compound FC-203 effectively reduces the intensity of the symptoms associated with uncontrolled protein aggregation in tissues of the nervous system in animals.

**Example 12. Determination of FC-203 effect on the formation of aggregates of FUS protein in cell cultures**

The ability of the testing compound to affect the formation and/or stability of aggregates of the pathogenic protein was assessed in cell cultures expressing aberrant forms of the human aggregation-prone FUS protein. Cell cultures of the human neuroblastoma cell line SH-SY5Y were transfected with plasmid encoding mutant human FUS proteins associated with some hereditary forms of Amyotrophic lateral sclerosis (ALS). These mutant isoforms exhibit increased aggregation tendency and form pathogenic protein inclusions. Such a model system reproduces crucial stages of the molecular pathology of proteinopathies which are associated with disruption in the metabolism of these proteins. FC-203 was added right after transfection to the cell cultures in the culture medium, followed by the assessment of the number of FUS protein-formed inclusions. The aggregates of FUS protein in the control group were gradually accumulated leading to the death of cells. However, the addition of compound FC-203 significantly inhibited the process of the formation of protein aggregates.

Fig.11 shows FUS-positive aggregates are localized in cytoplasm of the control cell culture, whereas the FUS staining in treated cells is diffused. This fact support the hypothesis that testing compound inhibits the formation of FUS-protein aggregates.

**Example 13. Determination of FC-203 effect on the formation of aggregates of TDP-43 protein in cell cultures**

Another protein that was used to study the unexpectedly found properties of FC-203 to inhibit the formation of cytoplasmic protein aggregates was TDP-43 protein. Similarly to FUS protein, it is a DNA/RNA-binding protein and has similar structure and functions in regulation of RNA metabolism. Moreover, it was found to play a role in some forms of ALS and Frontotemporal degeneration where revealed gene mutations lead to the change in the primary sequence of the encoded protein. A plasmid of a truncated human TDP-43 gene encoding an aberrant form of this protein characterized by high aggregation properties was cloned into the expression vector and transfected into human neuroblastoma cells SH-SY5Y as described in the previous example. The formation of inclusions was assessed after 18 hours in the cytoplasm. As a result of treatment with testing compound, the number of aggregates was significantly reduced, which was also due to decreased aggregate formation of aberrant TDP-43 protein.

Fig.12 shows that TDP-43-positive aggregates are localized in the cytoplasm of the control group, whereas treated cells demonstrate a reduced number of stained TDP-43-positive aggregates. Thus, given specific aggressive properties of the used aberrant form of TDF-43 protein, FC-203 prevents aggregation of TDP-43.

The following aspect of the invention is a pharmaceutical agent improving cognitive functions and memory, comprising an effective amount of a compound of formula (I) and a pharmaceutically acceptable carrier.

The pharmaceutical agent according to the invention is prepared by well-known methods and comprises a pharmaceutically effective amount of an active agent which is a compound of formula (I) (further referred herein as "active agent"), wherein the effective amount is generally from 1 to 20 wt.%, or from 1 to 20 mg in a dosage form in combination with one or more pharmaceutically acceptable additives such as diluents, binders, disintegrants, absorbents, aromatizers, and flavoring agents. According to known methods, the pharmaceutical compositions can be presented in various liquid or solid forms. Examples of solid dosage forms include, for example, tablets, pills, gelatinized capsules, etc.

The compositions are usually prepared by standard procedures providing mixing the active agent with a liquid or finely powdered solid carrier.

The tableted composition according to the invention comprises from 1 to 20 wt.% of the active agent and a carrier or an excipient such as: a) diluents: beet sugar, lactose, glucose, sodium chloride, sorbitol, mannitol, glycol, calcium phosphate; b) binders: magnesium aluminium silicate, starch paste, gelatin, tragacanth, methylcellulose, carboxymethyl cellulose, and polyvinylpyrrolidone. c) disintegrants: dextrose, agar, alginic acid or a salt thereof, starch, and tween.

EXAMPLE 1

A 100 mg tablet comprises 5 mg of the preparation

| | |
|---|---|
| Preparation | 5 mg |
| Lactose | 50.0 mg |
| Alginic acid | 20.0 mg |
| Citric acid | 5.0 mg |
| Tragacanth | 20.0 mg |

A tablet may be produced by pressing or molding the active agent with one or more additives.

The pressed tablets are produced on a special apparatus. A free-form of the active agent such as powder or granules in an amount of 50 mg (the amount of the compound required to produce 10000 tablets) is mixed with a binder (tragacanth, 200 g), and stirred with a diluent (lactose, 500 g), followed by the addition of a disintegrant (alginic acid, 200 g) and a citric acid (50 g) to the mixture.

Gelatinized capsules comprise further colorants and stabilizers. Suitable colorants are tetrazine and indigo; stabilizers can be selected from sodium metabisulphite and sodium benzoate. The claimed gelatinized capsules comprise from 1 to 20% of the active agent.

EXAMPLE 2

500 mg capsules comprising 20 mg of the preparation.

| | |
|---|---|
| Preparation | 20 mg |
| Glycerin | 100.0 mg |
| Sugar syrup | 319.0 mg |
| Mint oil | 40.0 mg |
| Sodium benzoate | 10.0 mg |
| Ascorbic acid | 5.0 mg |
| Tetrazine | 5.0 mg |

200 g of the active agent (the number of the compound required to produce 10000 tablets) are finely powdered and mixed with glycerin (1000 g) and sugar syrup (3190 g) in a mixer. After mixing, mint oil (400 g), sodium benzoate (100 g), ascorbic acid (50 g) and tetrazine (50 g) are added to the mixture. Gelatinized capsules are produced by a drop method. This method allows drop-wise dosing simultaneously both the drug solution and gelatinized heated mass (gelatin, 900 g) into liquid paraffin, thereby forming seamless spherical gelatinized capsules filled with a ready-to-use pharmaceutical mixture comprising 20 mg of the active agent.

According to the invention, a method for improving cognitive functions and memory comprises administering to a patient a pharmacological agent comprising an effective amount of fluorine-containing substituted 5-[2-(pyrid-3-yl)-ethyl]-2,3,4-tetrahydro-1H-pyrido[4,3-b]indoles of formula (I) in a dose of 1 to 150 mg at least once a day over a time required for a therapeutic effect.

A prescribed administered dose of the active agent - a compound of formula (I), varies depending on a plurality of factors such as age, sex, body weight, a particular compound to be administered, a mode of administration, a prescribed preparative form of the active agent.

As can be seen from the provided examples, the claimed invention addresses the problem of broadening the range of agents reducing, in the nervous system, uncontrolled protein aggregation resulting in the development of destructive processes in the central and peripheral nervous system.

Table 1. Yields, melting points of the synthesized compounds

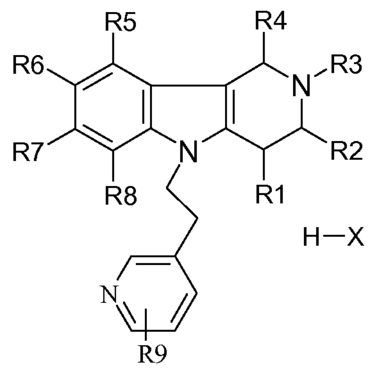

| No | R1 | R2 | R3 | R4 | R5 | R6 | R7 | R8 | R9 | X | Yield, % | Melting point, °C |
|---|---|---|---|---|---|---|---|---|---|---|---|---|
| 1 | H | H | Me | H | H | H | H | H | F | - | 68 | 97-99 |

(continued)

| No | R1 | R2 | R3 | R4 | R5 | R6 | R7 | R8 | R9 | X | Yield, % | Melting point, °C |
|---|---|---|---|---|---|---|---|---|---|---|---|---|
| 2 | H | H | Me | H | H | Me | H | H | F | - | 72 | 103-104 |
| 3 | H | H | Me | H | H | Br | H | H | F | - | 69 | 100-102 |
| 4 | H | H | Me | H | H | Cl | H | H | F | - | 73 | 111-112 |
| 5 | H | H | Me | H | H | F | H | H | F | - | 70 | 92-94 |
| 6 | H | H | Me | H | H | MeO | H | H | F | - | 65 | 105-107 |
| 7 | H | H | Et | H | H | Me | H | H | F | - | 76 | 93-95 |
| 8 | H | H | Me | H | H | H | H | H | F | Cl | 85 | 235-237 |
| 9 | H | H | Me | H | H | Me | H | H | F | Cl | 91 | 238-240 |
| 10 | H | H | Me | H | H | Br | H | H | F | Cl | 81 | 213-215 |
| 11 | H | H | Me | H | H | Cl | H | H | F | Cl | 88 | 252-254 |
| 12 | H | H | Me | H | H | F | H | H | F | Cl | 90 | 237-239 |
| 13 | H | H | Me | H | H | MeO | H | H | F | Cl | 82 | 214-216 |
| 14 | H | H | Me | H | H | $CF_3O$ | H | H | F | Cl | 77 | 228-230 |
| 15 | H | H | Et | H | H | H | H | H | F | Cl | 87 | 220-222 |
| 16 | H | H | Et | H | H | Me | H | H | F | Cl | 93 | 216-218 |
| 17 | H | H | Et | H | H | Br | H | H | F | Cl | 86 | 229-230 |
| 18 | H | H | Et | H | H | Cl | H | H | F | Cl | 82 | 241-243 |
| 19 | H | H | Et | H | H | F | H | H | F | Cl | 84 | 231-233 |
| 20 | H | H | Et | H | H | MeO | H | H | F | Cl | 80 | 202-204 |
| 21 | H | H | Et | H | H | $CF_3O$ | H | H | F | Cl | 74 | 201-203 |
| 22 | H | H | Me | H | H | Me | H | H | F | Br | 82 | 206-208 |
| 23 | H | H | Me | H | H | H | H | H | $CF_3$ | - | 67 | 87-89 |
| 24 | H | H | Me | H | H | Me | H | H | $CF_3$ |  | 72 | 94-96 |
| 25 | H | H | Me | H | H | Cl | H | H | $CF_3$ |  | 65 | 84-86 |
| 26 | H | H | Me | H | H | F | H | H | $CF_3$ |  | 69 | 85-87 |
| 27 | H | H | Me | H | H | MeO | H | H | $CF_3$ |  | 63 | 91-93 |
| 28 | H | H | Me | H | H | F | H | F | $CF_3$ |  | 76 | 101-103 |
| 29 | H | H | Et | H | H | Cl | H | H | $CF_3$ |  | 78 | oil |
| 30 | H | H | Me | H | H | H | H | H | $CF_3$ | Cl | 89 | 111-113 |
| 31 | H | H | Me | H | H | Me | H | H | $CF_3$ | Cl | 92 | 115-116 |
| 32 | H | H | Me | H | H | Cl | H | H | $CF_3$ | Cl | 95 | 117-119 |
| 33 | H | H | Me | H | H | F | H | H | $CF_3$ | Cl | 87 | 112-114 |
| 34 | H | H | Me | H | H | MeO | H | H | $CF_3$ | Cl | 91 | 132-134 |
| 35 | H | H | Me | H | H | F | H | F | $CF_3$ | Cl | 90 | 121-122 |
| 36 | H | H | Et | H | H | Cl | H | H | $CF_3$ | Cl | 88 | 57-59 |

Table 2. Data of $^{1}H$ and $^{19}F$ NMR spectra of the synthesized compounds

| No | $^{1}H$ NMR, δ, m.d. (J/Hz) | $^{19}F$ NMR, δ, m.d., (J/Hz) |
|---|---|---|
| 1 | 2.58 (s+t, 5H), 2.77 (t, 2H, *J*=6.1 Hz), 3.08 (t, 2H, *J*=3.1 Hz), 3.70 (s, 2H), 4.45 (t, 2H, *J*=3.1 Hz), 6. 93 (d, 1H *J*=7.2 Hz), 7. 05-7.33 (m, 4H), 7.47 (d, 1H *J*=3.1 Hz), 8.20 (s, 1H), 8.38 (d, 1H, *J*=3.1 Hz) | -47.12 (d, J=9 Hz) |
| 2 | 2.44 (s, 3H), 2.58 (s+t, 5H), 2.71 (t, 2H, *J*=6.1 Hz), 3.02 (t, 2H, *J*=6.1 Hz), 3.63 (s, 2H), 4.21 (t, 2H, *J*=6.1 Hz), 6.88 (d, 1H, *J*=7.1 Hz), 6.98 (d, 1H, *J*=7.1 Hz), 7.10 (d, 1H *J*=7.1 Hz), 7.21 (s, 1H), 8.13 (s, 1H), 8.32 (d, 1H, *J*=3 Hz) | -47.46 (d, *J*=9 Hz) |
| 3 | 2.53 (s+t, 5H), 2.76 (t, 2H, *J*=6.2 Hz), 3.06 (t, 2H, *J*=6.2 Hz), 3.65 (s, 2H), 4.26 (t, 2H, *J*=6.2 Hz), 6.88 (d, 1H, *J*=6.8 Hz), 7.07 (d, 1H, *J*=6.8 Hz), 7.26 (d, 1H *J*=6.8 Hz), 7.57 (s, 1H), 8.15 (s, 1H), 8.37 (d, 1H, *J*=3 Hz) | -49.02 (d, *J*=9.5 Hz) |
| 4 | 2.48 (s+t, 5H), 2.57 (t, 2H, *J*=6.2 Hz), 2.92 (t, 2H, *J*=6.2 Hz), 3.45 (s, 2H), 4.11 (t, 2H, *J*=6.2 Hz), 6.6 (d, 1H, *J*=7.2 Hz), 6.97 (s, 2H), 7.28 (s, 1H), 8.01 (s, 1H), 8.25 (d, 1H, *J*=3 Hz) | -48.22 (d, *J*=9.7 Hz) |
| 5 | 2.56 (s+t, 5H), 2.76 (t, 2H, *J*=5.9 Hz), 3.07 (t, 2H, *J*=5.9 Hz), 3.64 (s, 2H), 4.27 (t, 2H, *J*=5.9 Hz), 6.90 (m, 2H), 7.11 (m, 2H), 8.17 (s, 1H), 8.37 (d, 1H, *J*=3 Hz) | -46.13 (dt, 1F, *J*=4.2 Hz, *J*=9.7 Hz),-49.11 (d, 1F, *J*=9 Hz) |
| 6 | 2.54 (s+t, 5H), 2.75 (t, 2H, *J*=5.9 Hz), 3.06 (t, 2H, *J*=5.9 Hz), 3.67 (s, 2H), 3.90 (s, 3H), 4.26 (t, 2H, *J*=5.9 Hz), 6.83 (d, 1H, *J*=6.9 Hz), 6.92 (s, 1H), 7.13 (d, 1H, *J*=5.9 Hz), 7.30 (s, 2H), 8.18 (s, 1H), 8.36 (d, 1H, *J* =3 Hz) | -49.11 (d, *J*=9 Hz) |
| 7 | 1.20 (t, 3H, *J*=6.2 Hz), 2.46 (s+t, 5H), 2.63 (m, 2H), 2.76 (m, 2H), 3.01 (t, 2H), 3.67 (s, 2H), 4.20 (t, 2H), 6.87 (d, 1H), 6.97 (s, 1H), 7.09 (d, 1H), 7.23 (1, 2H), 8.16 (s, 1H), 8.32 (d, 1H, J 3) | -48.22 (d, *J*=9 Hz) |
| 8 | 2.95 (d, 3H, *J*=4.9 Hz), 3.05-3.24 (m, 4H), 3.46 (m, 1H), 3.76 (m, 1H), 4.29 (m, 1H), 4.45 (t, 2H, *J*=4.9 Hz), 4.62 (m, 1H), 7.09 (m, 2H), 7.46 (m, 2H), 7.80 (d, 1H, *J*=9 Hz), 8.33 (s, 1H), 8.53 (d, 1H, *J*=3 Hz), 11.23 (br s, 1H) | -48.70 (d, *J*=9 Hz) |
| 9 | 2.92 (d, 3H, *J*=4.4 Hz), 3.07-3.26 (m, 4H), 3.43 (m, 1H), 3.70 (m, 1H), 4.24 (m, 1H), 4.38 (t, 2H, *J*=4.9 Hz), 4.55 (m, 1H), 6.95 (d, 1H, J=8.6 Hz), 7.21 (s, 1H), 7.34 (d, 1H, *J*=8.6 Hz), 7.88 (d, 1H, *J*=9.7 Hz), 8.36 (s, 1H), 8.58 (br s, 1H), 11.39 (br s, 1H) | -47.23 (d, *J*=9 Hz) |
| 10 | 2.92 (d, 3H, *J*=4.4 Hz), 3.07-3.26 (m, 4H), 3.44 (m, 1H), 3.75 (m, 1H), 4.24 (m, 1H), 4.43 (t, 2H, *J*=4.9 Hz), 4.58 (m, 1H), 7.22 (d, 1H, *J*=8.6 Hz), 7.44 (d, *J*=8.6, Hz), 7.69 (s, 1H), 7.76 (d, 1H, *J*=9.5 Hz), 8.28 (s, 1H), 8.48 (d, 1H, *J*=3 Hz), 11.39 (br s, 1H) | -49.21 (d, *J*=9.5 Hz) |
| 11 | 2.92 (d, 3H, *J*=4.4 Hz), 3.07-3.26 (m, 4H), 3.44 (m, 1H), 3.75 (m, 1H), 4.24 (m, 1H), 4.43 (t, 2H, *J*=4.9 Hz), 4.55 (m, 1H), 7.10 (dd, 1H, J=2.3 Hz, *J*=8.8 Hz), 7.48 (d, *J*=8.8 Hz), 7.55 (d, 1H, *J*=2.3 Hz), 7.89 (d, 1H, *J*=9.7 Hz), 8.37 (s, 1H), 8.58 (d, 1H, *J*=3), 11.51 (br s, 1H) | -47.98 (d, *J*=9.7 Hz) |
| 12 | 2.95 (d, 3H, *J*=4.9 Hz), 3.07-3.24 (m, 4H), 3.46 (m, 1H), 3.76 (m, 1H), 4.23 (m, 1H), 4.43 (t, 2H, *J*=4.9 Hz), 4.54 (m, 1H), 6.96 (ddd, 1H, *J*=2.7 Hz, *J*=9.1 Hz, *J*=9.7 Hz), 7.27 (dd, 1H, *J*=2.7 Hz, *J*=9.7), 7.47 (dd, *J*=9.1 Hz, *J*= 4.2 Hz), 7.78 (d, 1H, *J*=9 Hz), 8.30 (s, 1H), 8.51 (d, 1H, *J*=3 Hz), 11.23 (br s, 1H) | -46.51 (dt, 1F, *J*=4.2 Hz, *J*=9.7 Hz),-49.00 (d, 1F, *J*=9 Hz) |
| 13 | 2.94 (d, 3H, *J*=4.4 Hz), 3.07-3.26 (m, 4H), 3.42 (m, 1H), 3.73 (m, 1H), 3.77 (s, 3H); 4.24 (m, 1H), 4.37 (t, 2H, *J*=4.9 Hz), 4.55 (m, 1H), 6.98 (d, 1H, *J*=2.3 Hz), 7.10 (dd, 1H, *J*=2.3 Hz, *J*=8.6 Hz), 7.36 (d, *J*=8.6 Hz), 7.72 (d, 1H, *J*=9.7 Hz), 8.29 (s, 1H), 8.48 (d, 1H, *J*=3 Hz), 10.96 (br s, 1H) | -49.36 (d, *J*=9 Hz) |
| 14 | 2.95 (d, 3H, *J*=4.0 Hz), 3.07-3.24 (m, 4H), 3.46 (m, 1H), 3.76 (m, 1H), 4.23 (m, 1H), 4.43 (m, 2H), 4.54 (m, 1H), 7.07 (dd, 1H, J=2.0 Hz, *J*=9.3 Hz), 7.56 (d, 1H, *J*=9.3 Hz), 7.82 (d, 1H, *J*=9.7 Hz), 8.36 (s, 1H), 8.53 (d, 1H, *J*=2 Hz), 11.43 (br s, 1H) | 20.81 (s, 3F), -48.63 (d, 1F, *J*=8.5 Hz) |

(continued)

| No | $^1$H NMR, δ, m.d. (J/Hz) | $^{19}$F NMR, δ, m.d., (J/Hz) |
|---|---|---|
| 15 | 1.40 (t, 3H, *J*=4.9 Hz), 3.07-3.24 (m, 4H), 3.29 (m, 2H), 3.46 (m, 1H), 3.76 (m, 1H), 4.29 (m, 1H), 4.25 (m, 2H), 4.63 (m, 1H), 7.07 (m, 2H), 7.47 (m, 2H), 7.76 (d, 1H, *J*=9.0 Hz), 8.30 (s, 1H), 8.48 (d, 1H, *J*=3.0 Hz), 11.00 (br s, 1H) | -49.07 (d, *J*=9 Hz) |
| 16 | 1.38 (t, 3H, *J*=4.9 Hz), 2.38 (s, 3H), 3.06-3.24 (m, 4H), 3.27 (m, 2H), 3.45 (m, 1H), 3.78 (m, 1H), 4.26 (m, 2H), 4.30 (m, 1H), 4.58 (m, 1H), 6.95 (d, 1H, *J*=8.0 Hz), 7.27 (s, 1H), 7.34 (d, *J*=8.0 Hz), 7.75 (d, 1H, *J*=9.0 Hz), 8.27 (s, 1H), 8.49 (d, 1H, *J*=3.1 Hz), 10.87 (br s, 1H) | -49.13 (d, *J*=9 Hz) |
| 17 | 1.36 (t, 3H, *J*=5.1 Hz), 3.07 (m, 2H), 3.05-3.31 (m, 4H), 3.40 (m, 1H), 3.75 (m, 1H), 4.22 (m, 1H), 4.66 (m, 1H), 7.10 (d, 1H, *J*=8.0 Hz), 7.48 (d, *J*=8.0 Hz), 7.59 (s, 1H), 8.11 (d, 1H, J=9.0 Hz), 8.44 (s, 1H), 8.72 (br s, 1H), 10.54 (br s, 1H) | -49.01 (d, *J*=9 Hz) |
| 18 | 1.37 (t, 3H, *J*=4.9 Hz), 3.08 (m, 2H), 3.07-3.30 (m, 4H), 3.44 (m, 1H), 3.80 (m, 1H), 4.24 (m, 1H), 4.65 (m, 1H), 7.09 (d, 1H, *J*=8.0 Hz), 7.46 (d, *J*=8.0 Hz), 7.59 (s, 1H), 8.09 (d, 1H, *J*=9.0 Hz), 8.45 (s, 1H), 8.70 (br s, 1H), 10.56 (br s, 1H) | -48.93 (d, J=9 Hz) |
| 19 | 1.24 (t, 3H, *J*=4.9 Hz), 2.68 (q, 2H, *J*=4,9 Hz), 2.55 (m, 2H), 2.80 (m, 2H), 3.06 (t, 4H, *J*=4.9 Hz), 3.67 (s, 2H), 4.26 (t, 2H, *J*=4.9 Hz), 6.91 (m, 2H), 7.12 (m, 2H), 8.16 (s, 1H), 8.38 (d, 1H, *J*=3.3 Hz), 11.23 (br s, 1H) | -47.37 (dt, 1F, *J*=4.4 Hz, *J*=9.7 Hz), - 49.10 (d, 1F, *J*=9 Hz) |
| 20 | 1.35 (t, 3H, *J*=4.9 Hz), 3.09 (m, 2H), 3.08-3.30 (m, 4H), 3.43 (m, 1H), 3.62 (s, 3H); 3.88 (m, 1H), 4.25 (m, 1H), 4.65 (m, 1H), 7.08 (d, 1H, *J*=8.0 Hz), 7.44 (d, 1H, *J*=8.0 Hz), 7.59 (s, 1H), 8.11 (d, 1H, *J*=9.0 Hz), 8.49 (s, 1H), 8.71 (br s, 1H), 10.53 (br s, 1H) | -48.93 (d, *J*=9 Hz) |
| 21 | 1.39 (t, 3H, *J*=4.9 Hz), 3.06 (m, 2H), 3.07-3.31 (m, 4H), 3.45 (m, 1H), 3.82 (m, 1H), 4.23 (m, 1H), 4.66 (m, 1H), 7.09 (d, 1H, J=8.0 Hz), 7.47 (d, 1H, *J*=8.0 Hz), 7.61 (s, 1H), 8.10 (d, 1H, *J*=9.0 Hz), 8.47 (s, 1H), 8.71 (br s, 1H), 10.55 (br s, 1H) | 20.89 (s, 3F), -48.55 (d, 1F, *J*=8.5 Hz) |
| 22 | 2.95 (d, 3H, *J*=4.4 Hz), 3.00-3.21 (m, 4H), 3.41 (m, 1H), 3.72 (m, 1H), 4.20 (m, 1H), 4.31 (t, 2H *J*=4.9 Hz), 4.52 (m, 1H), 6.98 (d, 1H, *J*=8.6 Hz), 7.29 (s, 1H), 7.3 (d, 1H, *J*=8.6 Hz), 7.92 (d, 1H, *J*=9,7 Hz), 8.41 (s, 1H), 8.62 (br s, 1H, *J* 3), 11.44 (br s, 1H) | -48.06 (d, J=9 Hz) |
| 23 | 2.25 (t, 2H, *J*=4.9 Hz), 2.39 (s, 3H), 2.55 (t, 2H, *J*=4.9 Hz), 2.99 (t, 2H, *J*=4.9 Hz), 3.50 (s, 2H), 4.13 (t, 2H, *J*=4.9 Hz), 7.00 (m, 4H), 7.28 (dd, 1H), 7,34 (d, 1H, *J*=8.1 Hz), 8.30 (s, 1H) | 9.24 s |
| 24 | 2.24 (t, 2H, *J*=4.8 Hz), 2.39 and 2,42 both (t, 6H, *J*=4.8 Hz), 2.53 (t, 2H, *J*=4.8 Hz), 3.03 (t, 2H, *J*=4.8 Hz), 3.47 (s, 2H), 4.16 (t, 2H, *J*=4.8 Hz), 6.82 (d, 1H, *J*=8.2 Hz), 6.95 (m, 2H), 7.07 (s, 1H), 7.36 (d, 1H, *J*=8.2 Hz), 8.34 (s, 1H) | 9.59 s |
| 25 | 2.24 (t, 2H, *J*=4.8 Hz), 2.39 (s, 3H), 2.54 (t, 2H, *J*=4.8 Hz), 3.02 (t, 2H, *J*=4.8 Hz), 3.45 (s, 2H), 4.16 (t, 2H, *J*=4.8 Hz), 6.76 (td, 1H), 6.98 (m, 3H), 7.37 (d, 1H, *J*=8.0 Hz), 8.32 (s, 1H) | 9.62 s |
| 26 | 2.26 (t, 2H, *J*=4.8 Hz), 2.38 (s, 3H), 2.53 (t, 2H, *J*=4.8 Hz), 2.96 (t, 2H, *J*=4.8 Hz), 3.42 (s, 2H), 4.09 (t, 2H, *J*=4.8 Hz), 6.94 (m, 2H), 7.03 (d, 1H, *J*=8.1 Hz), 7.25 (s, 1H), 7.36 (d, 1H, *J*=8.1 Hz), 8.24 (s, 1H) | -45.12 (m, 1F), 9.51 (s, 3F) |
| 27 | 2.23 (t, 2H, *J*=4.9 Hz), 2.52 (s, 3H), 2.53 (t, 2H, *J*=4.8 Hz), 3.03 (t, 2H, *J*=4.8 Hz), 3.47 (s, 2H), 3.78 (s, 3H), 4.15 (t, 2H, *J*=4.8 Hz), 6.67 (m, 2H), 6.94 (m, 2H), 7.36 (d, 1H, *J*=8,0 Hz), 8.34 (s, 1H) | 9.42 s |

(continued)

| No | $^1H$ NMR, δ, m.d. (J/Hz) | $^{19}F$ NMR, δ, m.d., (J/Hz) |
|---|---|---|
| 28 | 2.33 (t, 2H, *J*=4.8 Hz), 2.22 (t, 2H, *J*=4.8 Hz), 2.55 (m, 4H), 2.98 (t, 2H, *J*=4.9 Hz), 3.48 (s, 2H), 4.13 (t, 2H, *J*=4.8 Hz), 6.95 (m, 3H), 7.34 (m, 2H), 8.35 (s, 1H) | -57.24 (m, 1F), -44.05 (m, 1F), 9.09 (s 3F) |
| 29 | 1.13 (t, 1H, *J*=4.8 Hz), 2.39 (s, 3H), 2.54 (t, 2H, *J*=4.8 Hz), 3.02 (t, 2H, *J*=4.8 Hz), 3.45 (s, 2H), 4.16 (t, 2H, *J*=4.8 Hz), 6.76 (td, 1H, *J*=4.8 Hz, *J*= 6.5 Hz), 6.98 (m, 3H), 7.37 (d, 1H, *J*=8.1 Hz), 8.32 (s, 1H) | 9.53 s |
| 30 | 2.76 (s, 3H), 2.99 (m, 4H), 3.52 (m, 2H), 3.92 - 4, 55 (m, 4H), 6,91 (m, 1H), 7,26 (m, 2H), 7.60 (m, 3H), 8.39 (m, 1H), 11.27 (br s, 1H) | 11.47 s |
| 31 | 2.38 (s, 3H), 2.92 (s, 3H), 3.11 (m, 2H), 3.61 (m, 4H), 4.07 - 4.76 (m, 4H), 6.94 (d, 1H, *J*=6.8 Hz), 7.21 (s, 1H), 7.23 (d, 1H, *J*=6.8 Hz), 7.82 (m, 2H), 8.54 (s, 1H), 11,13 (br s, 1H) | 11.39 s |
| 32 | 2.59 (s, 3H), 2.80 (m, 2H), 3.13 (m, 4H), 4.12 (m, 4H), 6.74 (d, 1H, *J*=6.9 Hz), 7.05 (m, 1H), 7.20 (s, 1H), 7.47 (m, 2H), 8.21 (s, 1H), 11.27 (br s, 1H) | 11.54 s |
| 33 | 2.52 (s, 3H), 2.91 (m, 3H), 3.11 (m, 3H), 4.17 - 4.64 (m, 4H), 6.92 (m, 1H), 7.25 (d, 1H, *J*=7.1 Hz), 7.39 (m, 1H), 7.76 (m, 1H), 7.86 (m, 1H), 8.54 (s, 1H), 11.43 (br s, 1H) | -44.59 (m, 1F), 11.49 (s, 3F) |
| 34 | 2.92 (d, 3H, *J*=3.8 Hz), 3.11 (m, 2H), 3.62 (m, 4H), 3.78 (s, 3H); 4.13 - 4.68 (m, 4H), 6.74 (dd, 1H, *J*=2.7 Hz, *J*=6.8 Hz), 6.99 (d, 1H, *J*=6.8 Hz), 7.33 (d, 1H, *J*=6.8 Hz), 7.83 (m, 2H), 8.54 (s, 1H), 11.04 (br s, 1H) | 11.53 s |
| 35 | 2.54 (s, 3H), 2.77 (m, 4H), 3.32 (m, 1H), 3.88 (m, 2H), 4.11 (m, 3H), 6.56 (t, 1H, *J*=6.7 Hz), 6.79 (d, 1H, *J*=6.7 Hz), 7.40 (m, 2H), 8.08 (s, 1H), 11.09 (br s, 1H) | -57.48 (m, 1F), -44.12 (m, 1F), 11.61 (s, 3F) |
| 36 | 1.37 (t, 3H, *J*=5.1 Hz), 3.12 (m, 2H), 3.10-3.35 (m, 4H), 3.42 (m, 1H), 3.75 (m, 1H), 4.23 (m, 1H), 4.71 (m, 1H), 7.10 (dd, 1H, *J*=2.5 Hz, *J*=6.8 Hz), 7.45 (d, 1H, *J*=6.8 Hz), 7.62 (d, 1H, *J*=6.8 Hz), 7.74 - 8.01 (m, 2H), 10.52 (br s, 1H) | 11.38 s |

## Claims

**1.** Fluorine-containing substituted 5-[2-(pyrid-3-yl)-ethyl]-2,3,4-tetrahydro-1H-pyrido[4,3-b]indoles, hydrochlorides and hydrobromides thereof, of general formula (I) as an effective agent for reducing uncontrolled protein aggregation in the nervous system

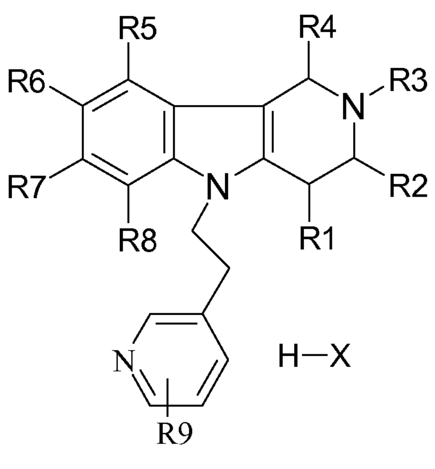

(I),

wherein

$R_1$=$R_2$=$R_4$ are H or Me; or $R_1$ + $R_2$ is -$CH_2$-$CH_2$-;
$R_3$ is H, ($C_1$-$C_6$)alkyl, ($C_2$-$C_6$)alkenyl, ($C_3$-$C_6$)alkynyl, ($C_1$-$C_6$)alkoxy($C_1$-$C_6$)alkyl, [F, Cl, Br, $NO_2$, ($C_1$-$C_6$)alkyl, ($C_1$-$C_6$)alkoxy, acyl, ($C_1$-$C_6$)alkylsulfonyl, ($C_1$-$C_6$)alkoxycarbonyl-substituted] aryloxy ($C_1$-$C_6$)alkyl, ($C_3$-$C_7$) cycloalkyl, [F, Cl, Br, $NO_2$, ($C_1$-$C_6$)alkyl, ($C_1$-$C_6$)alkoxy, acyl, ($C_1$-$C_6$)alkylsulfonyl, ($C_1$-$C_6$)alkoxycarbonyl-substituted]aryl, heteroaryl, ($C_1$-$C_6$)alkylsulfonyl, ($C_1$-$C_6$)alkoxycarbonyl, ($C_1$-$C_6$)acyl, [F, Cl, Br, $NO_2$, ($C_1$-$C_6$)alkyl, ($C_1$-$C_6$)alkoxy, acyl, ($C_1$-$C_6$)alkylsulfonyl, ($C_1$-$C_6$)alkoxycarbonyl-substituted]aroyl, heteroyl, N,N-dialkylcarbamoyl, N,N-dialkylaminosulfonyl;
$R_5$, $R_6$, $R_7$, $R_8$ = H, F, Cl, Br, CN, OH, $CF_3$, $CF_3O$, $CHF_2O$, $NO_2$, ($C_1$-$C_6$)alkyl, ($C_3$-$C_7$)cycloalkyl, ($C_1$-$C_6$)alkoxy, [F, Cl, Br, $NO_2$, ($C_1$-$C_6$)alkyl, ($C_1$-$C_6$)alkoxy, acyl, ($C_1$-$C_6$)alkylsulfonyl, ($C_1$-$C_6$)alkoxycarbonyl - substituted] aryl, [F, Cl, Br, $NO_2$, ($C_1$-$C_6$)alkyl, ($C_1$-$C_6$)alkoxy, acyl, ($C_1$-$C_6$)alkylsulfonyl, ($C_1$-$C_6$)alkoxycarbonyl - substituted]aryloxy, (Me, Cl, Br - substituted)pyridyl, ($C_1$-$C_6$)alkylsulfonyl, ($C_1$-$C_6$)alkoxycarbonyl, ($C_1$-$C_6$)acyl, [F, Cl, Br, $NO_2$, ($C_1$-$C_6$)alkyl, ($C_1$-$C_6$)alkoxy, acyl, ($C_1$-$C_6$)alkylsulfonyl, ($C_1$-$C_6$)alkoxycarbonyl - substituted] aroyl, N,N-dialkylcarbamoyl, N,N-dialkylaminosulfonyl;
R9 = F, 2F, $CHF_2$, $CClF_2$, $CF_3$;
X is absent or is Cl or Br.

**2.** A pharmacological agent for reducing uncontrolled protein aggregation in the nervous system, comprising an active agent and a pharmaceutically acceptable carrier, **characterized in that** the pharmacological agent comprises as the active agent an effective amount of a compound of formula (I).

**3.** A method for reducing uncontrolled protein aggregation in the nervous system, comprising administering to a patient a pharmacological agent comprising an effective amount of a compound of formula (I) in a dose of from 0.01 to 1.5 mg/kg of body weight once a day over a period of time required for a therapeutic effect.

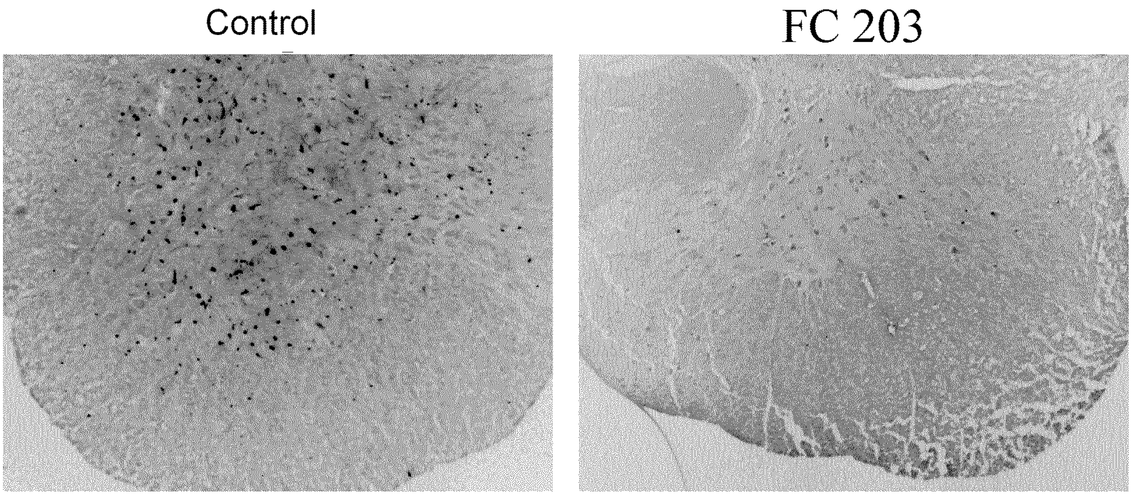


FIG. 1

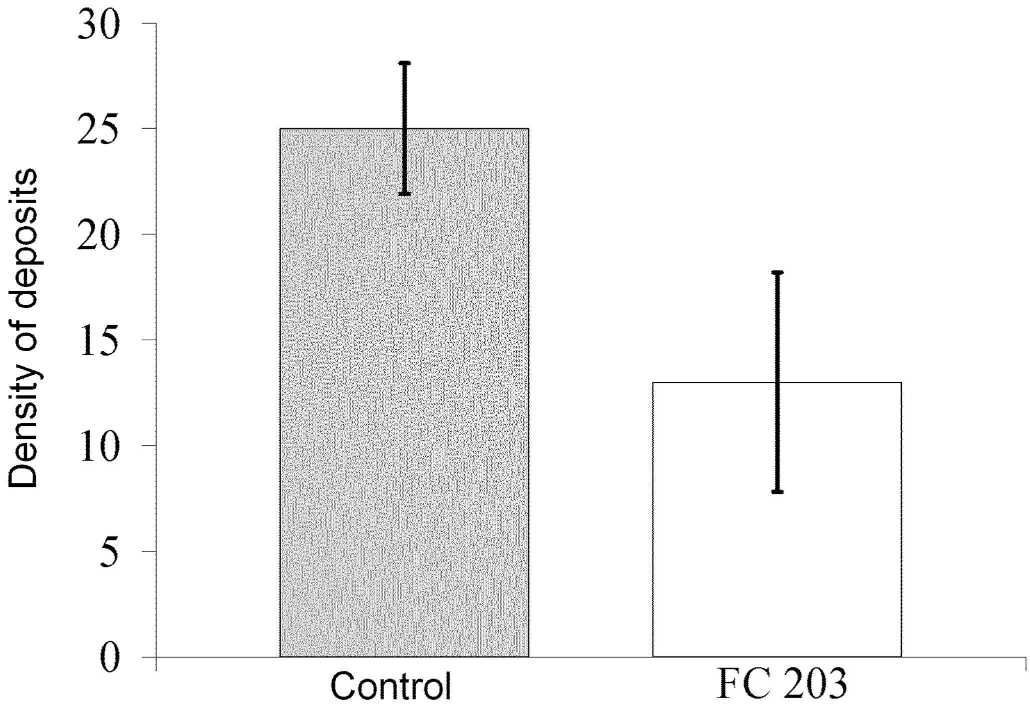


FIG. 2

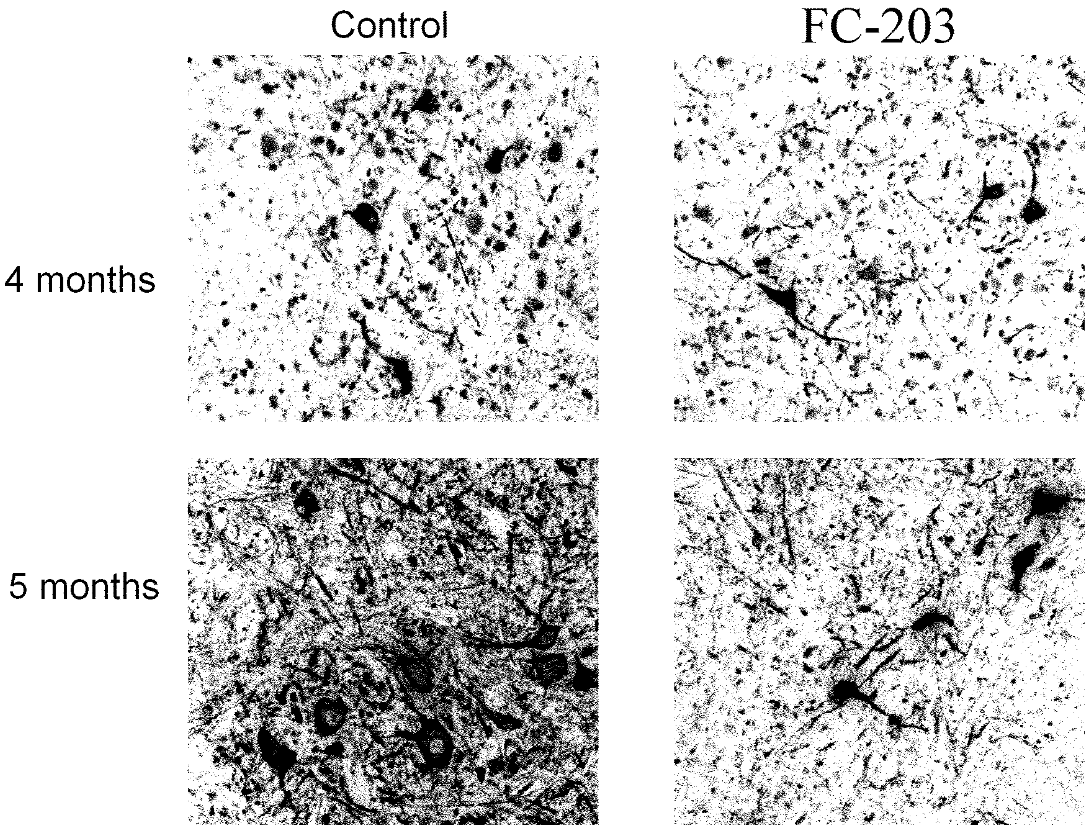


FIG. 3

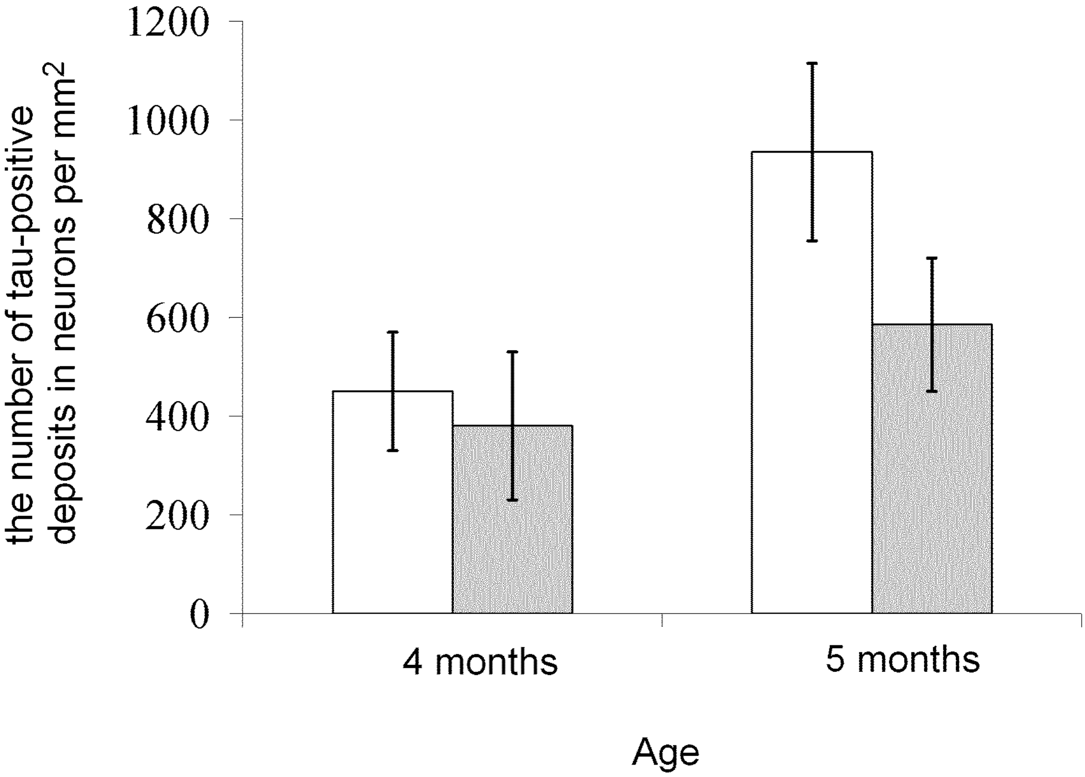
1200
1000
800
600
400
200
0
the number of tau-positive deposits in neurons per mm2
4 months
5 months
Age

FIG. 4

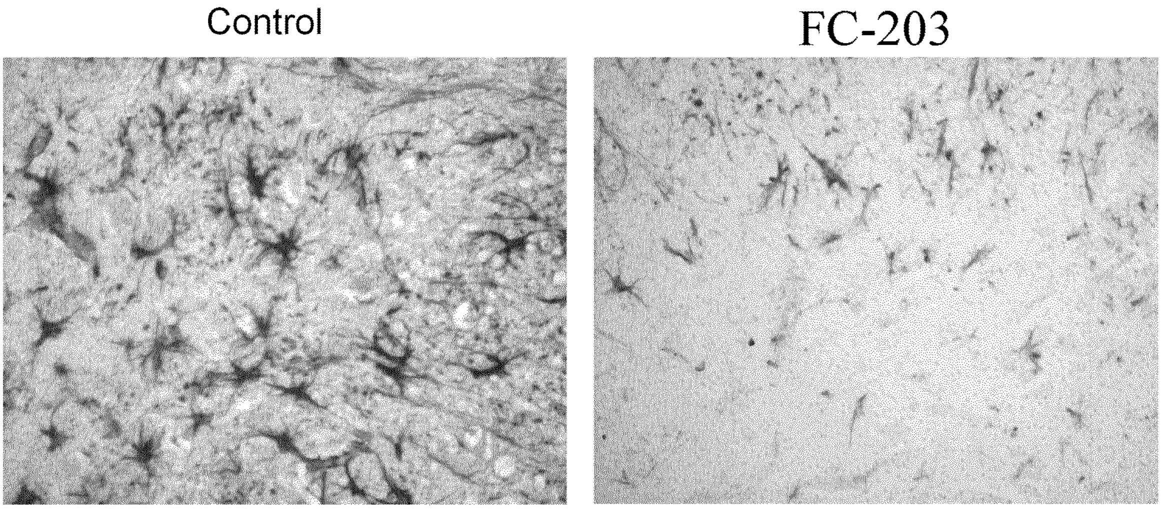


FIG. 5

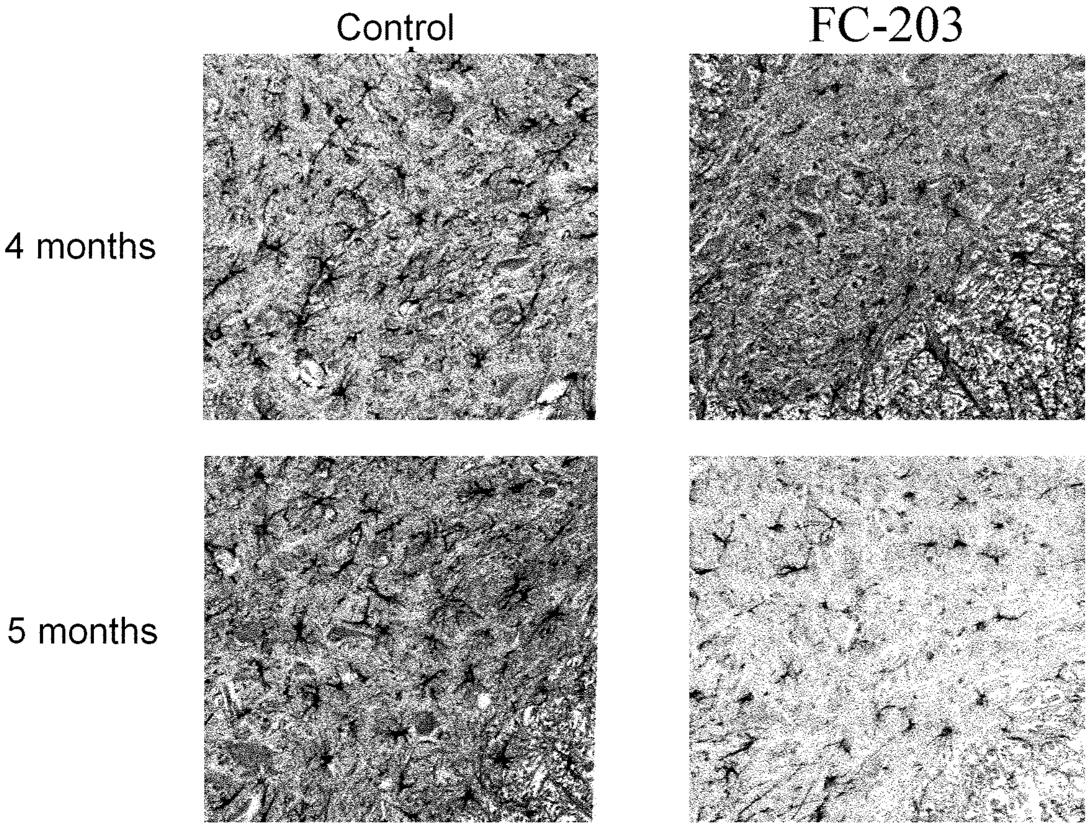


FIG. 6

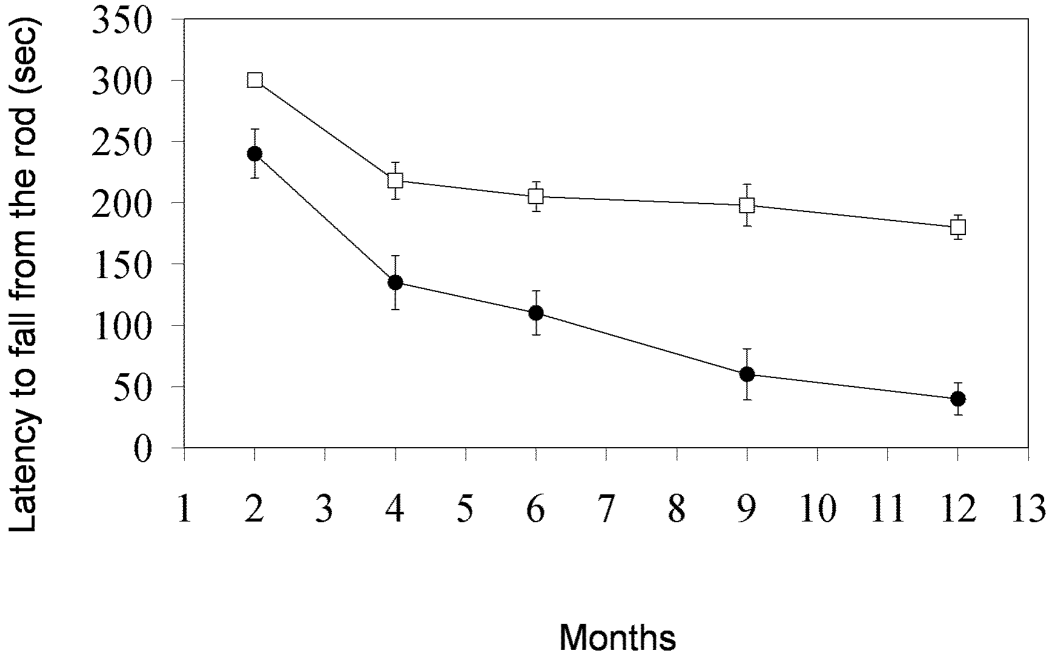
Latency to fall from the rod (sec)
350
300
250
200
150
100
50
0
1 2 3 4 5 6 7 8 9 10 11 12 13
Months

FIG. 7

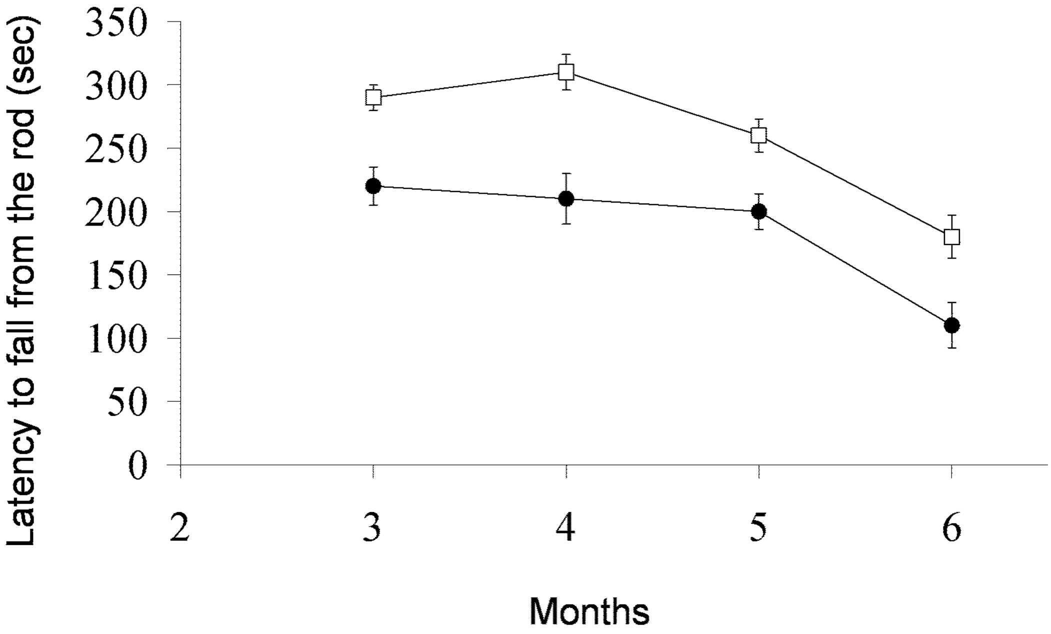
350
300
250
200
150
100
50
0
Latency to fall from the rod (sec)
2
3
4
5
6
Months

FIG. 8

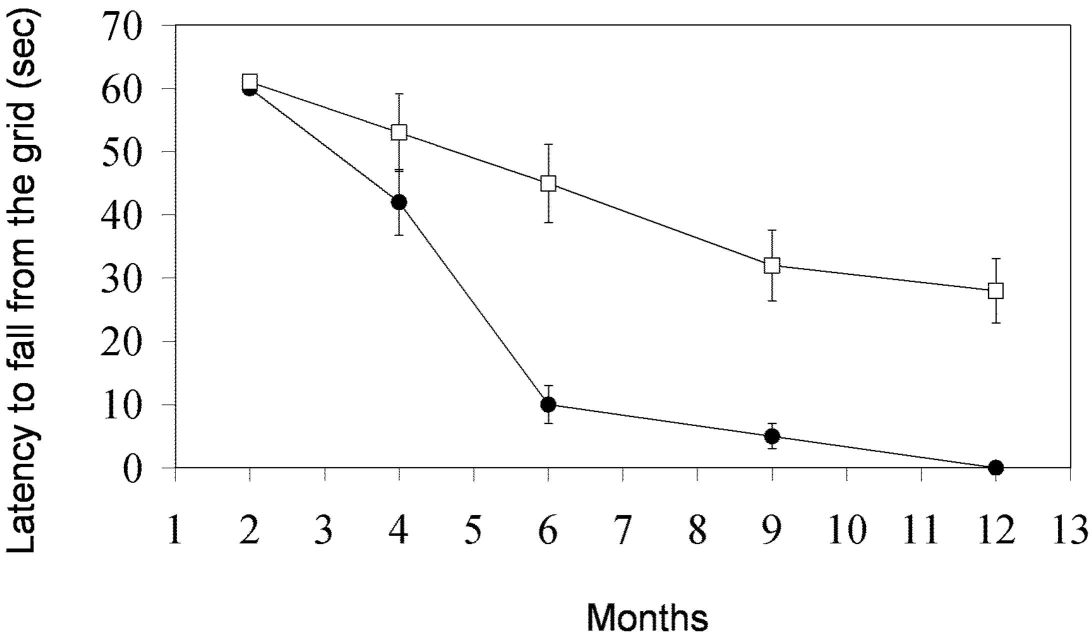
Latency to fall from the grid (sec)
70
60
50
40
30
20
10
0
1
2
3
4
5
6
7
8
9
10
11
12
13
Months

FIG. 9

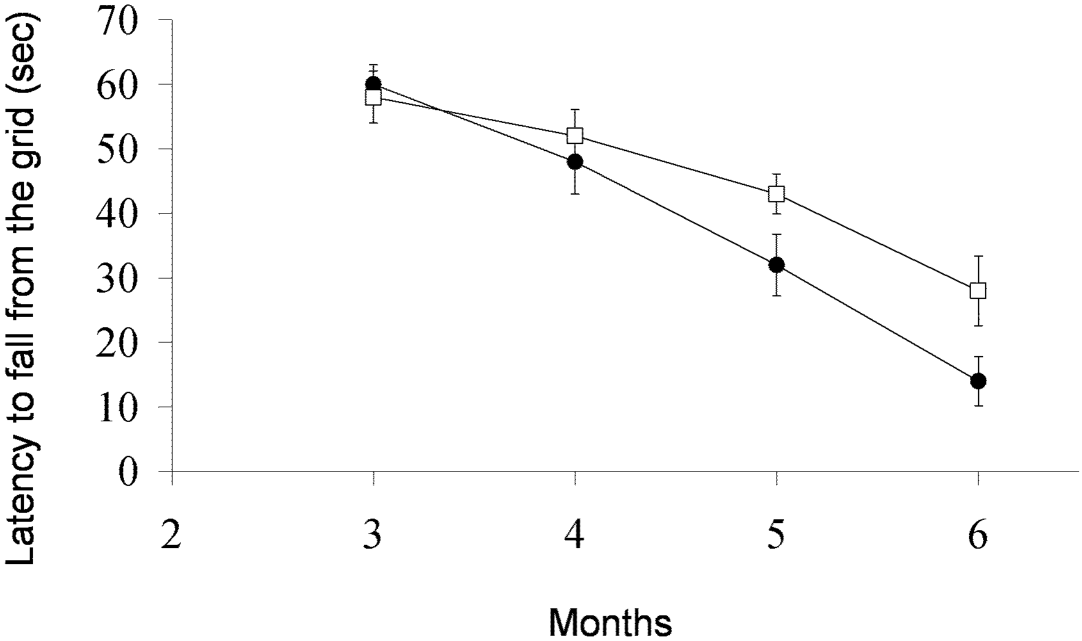
Latency to fall from the grid (sec)
70
60
50
40
30
20
10
0
2
3
4
5
6
Months

FIG. 10

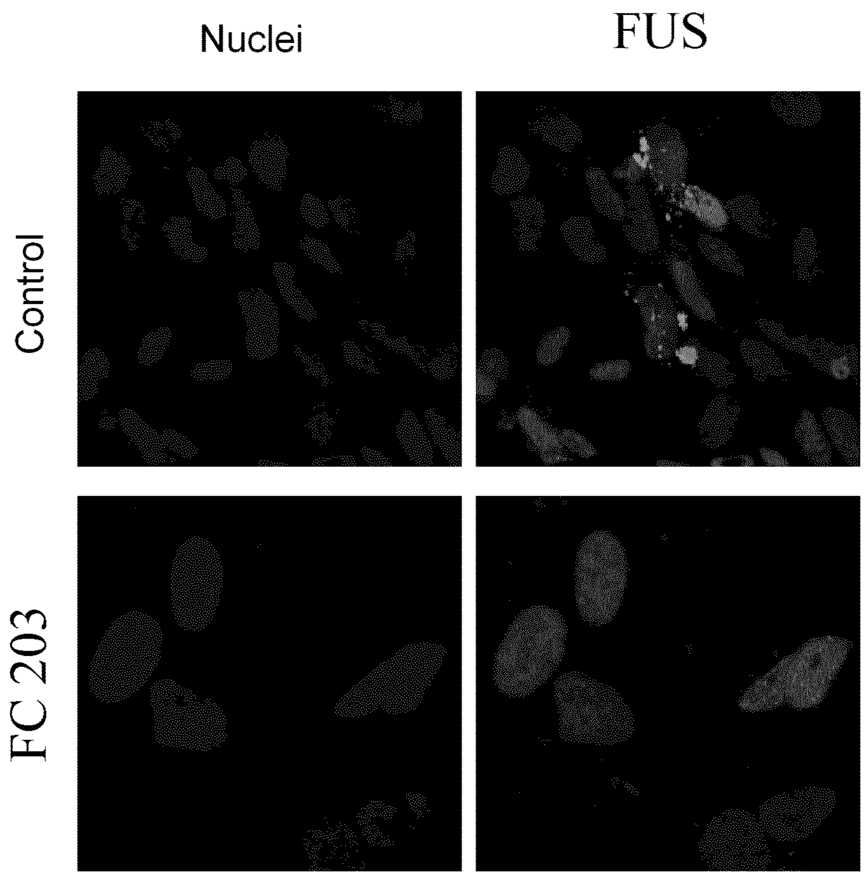


FIG. 11

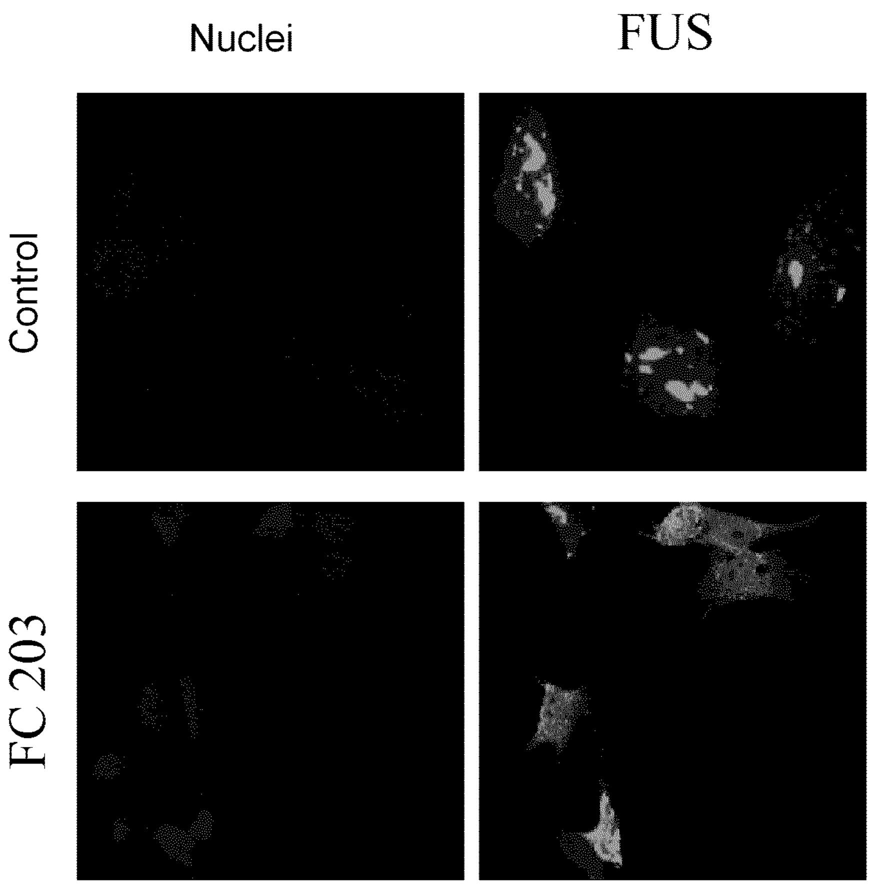


FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009038764 A1
- WO 2009055828 A1


### Non-patent literature cited in the description


- **WALKER L.C. ; IBEGBU C.C. ; TODD C.W. ; ROBINSONA H.L. ; JUCKERE M. ; ILLF H.L. ; GANDYG S.** *Biochemical Pharmacology,* 2005, vol. 69, 1001-1008
- **CHRISTENSEN D.D.** *CNS Spectrums,* 2007, vol. 12, 113-123
- **S.O. BACHURIN ; A.A. USTYUGOV ; O. PETERS ; T.A. SHELKOVNIKOVA ; V.L. BUCHMAN ; N.N. NINKINA.** *Doklady of the Russian Academy of Sciences,* 2009, vol. 428, 262-265